(19) Europäisches Patentamt
European Patent Office
Office européen des brevets

(11) EP 1 893 631 B1

(12) **EUROPEAN PATENT SPECIFICATION**

(45) Date of publication and mention of the grant of the patent:
**14.08.2013 Bulletin 2013/33**

(21) Application number: **06763772.8**

(22) Date of filing: **19.06.2006**

(51) Int Cl.:
***C07K 1/113*** *(2006.01)* ***C12N 9/64*** *(2006.01)*

(86) International application number:
**PCT/EP2006/063311**

(87) International publication number:
**WO 2006/134174 (21.12.2006 Gazette 2006/51)**

(54) **DIMERIC AND MULTIMERIC FVIIA COMPOUNDS**

DIMERE UND MULTIMERE FVIIA-VERBINDUNGEN

COMPOSÉS FVIIA DIMÈRES ET MULTIMÈRES

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.06.2005 EP 05105362**
**08.12.2005 EP 05111854**

(43) Date of publication of application:
**05.03.2008 Bulletin 2008/10**

(73) Proprietor: **Novo Nordisk Health Care AG**
**Thurgauerstrasse 36/38**
**8050 Zürich (CH)**

(72) Inventors:
- **ØSTERGAARD, Henrik**
**DK-3650 Ølstykke (DK)**
- **STENNICKE, Henning, Ralf**
**DK-2980 Kokkedal (DK)**

(74) Representative: **Nilsson, Karin Norvin**
**Novo Nordisk A/S**
**Corporate Patents**
**Novo Allé**
**2880 Bagsværd (DK)**

(56) References cited:
**WO-A-01/21661** **WO-A-02/077218**
**WO-A2-03/076461**

- **PERSSON E ET AL: "Augmented intrinsic activity of Factor VIIa by replacement of residues 305, 314, 337 and 374: evidence of two unique mutational mechanisms of activity enhancement" BIOCHEMICAL JOURNAL, THE BIOCHEMICAL SOCIETY, LONDON, GB, vol. 379, no. Pt 2, 15 April 2004 (2004-04-15), pages 497-503, XP002398830 ISSN: 0264-6021 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 893 631 B1

## Description

FIELD OF THE INVENTION

The present invention relates to novel FVIIa compounds, methods for their synthesis, pharmaceutical compositions comprising the novel compounds as well as their use in treatment of coagulation disorders.

BACKGROUND OF THE INVENTION

Coagulation factor VIIa (FVIIa) is the key initiator of haemostasis. It is a 50-kDa plasma protein with a functional circulatory half life around 1-3 hours. The zymogen, FVII, is a single-chain protein being catalytically inactive and may be converted into the catalytically active two-chain FVIIa by cleavage of an internal $Arg_{152}$-$Ile_{153}$ peptide bond. FVIIa is widely used as therapeutic protein for the treatment of various coagulation disorders that may be caused by clotting factor deficiencies or clotting factor inhibitors. FVIIa has also been used to control excessive bleeding occurring in subjects with a normally functioning blood clotting cascade. Such bleeding may for example be caused by a defective platelet function, thrombocytopenia, von Willebrands disease as well as during surgery and other forms of tissue damage.

For many of the therapeutic applications of FVIIa it is desirable to perform the treatment using FVIIa variants with improved properties relative to native FVIIa. One area where FVIIa variants with enhanced biological activity are desirable is the treatment of uncontrolled bleedings that are partially or completely refractory to conventional rFVIIa therapy due to suboptimal potency of FVIIa. Presently these so-called superactive FVIIa variants can be divided into two classes according to their mode of action. One group encompasses variants with improved proteolytic activity resulting from a shift in the conformational equilibrium of the protein towards the catalytically competent state, see e.g. Persson et al. (2001a), (2001b), (2002), (2004) and Soejima et al. (2002). The other group is mainly variants with optimized GLA domains exhibiting increased affinity for anionic phospholipids membranes (Shah et al. (1998), Nelsestuen et al. (2001) and Harvey et al. (2003)). It is believed that improved phospholipid-binding serves to localize and concentrate FVIIa onto the activated platelet membrane at the site of vascular injury thereby promoting haemostasis.

Covalent modification, e.g. by PEGylation or lipid attachment, has been successfully applied on several protein-based pharmaceutics to improve their pharmacokinetic and pharmacodynamic profiles. Conjugation via native or engineered cysteines provides an attractive means of site-specific modification due to the rarity of this amino acid on the surface of proteins, particularly those secreted by the cell, as well as the high selectivity of the thiol-coupling chemistry. The lack of free thiols in native Factor VIIa has led to the proposal that prolongation of the circulatory half life might be achieved by modification, e.g. PEGylation, of engineered solvent-exposed cysteines, see, e.g. WO 02/077218 A1 and WO 01/58935 A2.

Hence, WO 01/58935 A2 discloses FVIIa conjugates with non-polypeptide moieties and their preparation. It is, i.a., suggested that the non-polypeptide moiety is conjugated to the FVIIa polypeptide via a cysteine. Similarly, WO 02/077218 A1 discloses FVIIa polypeptide conjugates with chemical groups and their preparation. It is, i.a., suggested that the chemical group is conjugated to the FVIIa polypeptide via a cysteine.

In practice, however, this approach does not always warrant FVIIa compounds with the optimal match of pharmacokinetic and pharmacodynamic profiles, i.e. high activity, prolonged circulatory half-life etc.

Thus, there is a need for improved FVIIa compounds which exhibit high specific activity, prolonged plasma half-life as well as acceptable physico-chemical properties in relation to manufacturing, handling and formulation.

WO 03/076461 discloses dimeric Tissue Factor antagonists and their use in the treatment of e.g. vascular diseases and inflammatory diseases.

SE 9501285A discloses a process for the *in vitro* production of appropriately folded, biologically active disulfide-crosslinked proteins using a mixture of a protein disulfide oxidoreductase (e.g. protein disulfide isomerase (PDI)), a glutaredoxin and a redox buffer. The reference is focused on cysteines involved in intramolecular disulfide bonds.

SUMMARY OF THE INVENTION

In order to overcome the above-mentioned limitations of the known FVIIa compounds, the present invention now provides dimeric and multimeric FVIIa compounds comprising at least two FVIIa polypeptides covalently connected such as to retain the intrinsic catalytic activity of the FVIIa polypeptides. Such FVIIa compounds have surprisingly shown to be super-active and they are believed to exhibit enhanced membrane affinity resulting from the avidity effect arising when two or more FVIIa polypeptides are covalently linked.

In one aspect the present invention provides a FVIIa compound wherein two FVIIa polypeptides are covalently connected to form a dimeric FVIIa compound. In one embodiment the FVIIa polypeptides are covalently connected via engineered cysteines in said FVIIa polypeptides. In another embodiment two identical FVIIa polypeptides are covalently connected to form a dimeric FVIIa compound.

In another aspect the present invention provides a dimeric or multimeric FVIIa compound comprising a linker between the constituent FVIIa polypeptides.

The term "retain the intrinsic catalytic activity" refers to the property that the dimeric or multimeric FVIIa polypeptides have substantially the same peptidolytic activity as that of the constituent FVIIa polypeptides, e.g. an active site concentration which is at least 70%, preferably at least 80%, as measured by the assay disclosed herein.

BRIEF DESCRIPTION OF THE FIGURES

Figure 1: Identification of low-molecular weight thiols engaged in mixed disulfides with FVIIa 407C. Protein was incubated in the presence of TCEP and SBD-f to liberate and derivatize thiols attached to the engineered Cys407 in the preparation of FVIIa 407C. SBD-derivatized thiols were separated by reversed-phase HPLC and detected by fluorescence (excitation and emission wavelengths of 386 and 516 nm, respectively). HPLC traces of wild-type FVIIa and a series of low-molecular weight thiol compounds (10 pmol of each; denoted standard) are shown for comparison. Peaks marked with an asterisk arise from impurities in commercially available cysteamine. The two asterisks indicate a peak of unknown identity in the HPLC trace of FVIIa 407C. Percentages above each peak indicate the amount of a given thiol species (identified from its retention time) relative to the amount of FVIIa 407C analyzed. Based on retention times, it can be concluded that major thiols conjugated to FVIIa 407C are glutathione, cysteine, and homocysteine. Abbreviations: GSH (glutathione), y-GC (y-glutamylcysteine), CG (cysteinylglycine), Cys (cysteine), Hcy (homocysteine) and Cya (cysteamine).

Figure 2: Residual amidolytic activity of FVIIa incubated at pH 7 in the presence of varying concentrations of reduced and oxidized glutathione (given by the $[GSH]^2/[GSSG]$ ratio), 1 μM yeast glutaredoxin 1 (yGrx1p), and either no (open circles) or 25 mM p-aminobenzamidine (open squares). All samples were allowed to equilibrate for 3.5 hrs at 30°C before the amidolytic activity was measured. Amidolytic activities are normalized to 1 for fully active FVIIa.

Figure 3: Redox titration of the mixed-disulfide between FVIIa Cys407 and glutathione. FVIIa 407C was allowed to equilibrate in pH 7 buffer containing varying ratios of reduced and oxidized glutathione (given by the [GSH]/[GSSG] ratio) and 10 μM E.coli glutaredoxin 2 (Grx2). Following equilibration for 5 hrs at 30°C, free FVIIa 407C was detected and quantified by HPLC after alkylation with PEG5k-maleimide. Peak areas are normalized to 1 for fully 5k-PEGylated FVIIa.

Figure 4: Residual amidolytic activity (stippled lines) and fraction of FVIIa 407C with a free Cys407 thiol group (solid line) at equilibrium in a redox buffer consisting of 0.5 mM GSH, varying concentrations of GSSG, and either no (-PABA) or 25 mM p-aminobenzamidine (+PABA). Curves were drawn using Eq. 2 and 4, $K_{ox}$ values of 93 (-PABA) and 166 mM (+PABA), respectively, and a $K_{scox}$ value of 1.02. The shaded area indicates the concentration range of GSSG resulting in >90% residual activity and >90% free 407C thiol.

Figure 5: As Figure 4 with 0.25 mM GSH instead. The shaded area indicates the concentration range of GSSG resulting in >90% residual activity and >90% free FVIIa 407C thiol.

Figure 6: As Figure 4 with 1.0 mM GSH instead. The shaded area indicates the concentration range of GSSG resulting in >90% residual activity and >90% free FVIIa 407C thiol.

Figure 7: HPLC analysis of FVIIa 407C before (dotted line) and after selective reduction (dashed-dotted line), and after modification with PEG20k-maleimide (solid line). 407C, 407C-SR, and 407C-PEG20k indicate peaks representing free, low-molecular weight thiol-conjugated, and 20k-PEGylated FVIIa 407C, respectively. Asterisks indicate peaks of unknown identity probably representing hyper-PEGylated species. Peak integration yielded 89% free FVIIa 407C at the end of the reduction step versus 11% in the untreated material. After thiol alkylation, 85% of FVIIa 407C was converted into the mono-PEGylated species.

Figure 8: Reducing (right panel) and non-reducing (left panel) SDS-PAGE analysis of FVIIa 407C (lane A), FVIIa 407C-PEG5k (lane B), FVIIa 407C-PEG20k (lane C), FVIIa 407C-PEG40k (lane D), and FVIIa 407C-PEG3.4k-FVIIa 407C (lane E).

Figure 9: Reducing (right panel) and non-reducing (left panel) SDS-PAGE analysis of FVIIa 407C (lane A), FVIIa 407C-PEG3.4k-FVIIa 407C (lane B), FVIIa 407C-PEG20k-FVIIa 407C (lane C).

Figure 10: (A) Reducing SDS-PAGE analysis of FVIIa R396C treated with 2.5 (lane A) or 5.0 mM (lane B) triphenylphosphine-3,3',3" trisulfonic acid ($PPh_3S_3$) for 16.3 hrs at room temperature and then labelled with PEG20k-maleimide. Lane C contains untreated FVIIa as a reference. (B) Relative amidolytic activities of FVIIa R396C before (100% activity) and after 16.3 hrs incubation with $PPh_3S_3$.

DETAILED DESCRIPTION OF THE INVENTION

As mentioned above, the present invention provides dimeric and multimeric FVIIa compounds comprising at least two FVIIa polypeptides covalently connected such as to retain the intrinsic catalytic activity of the FVIIa polypeptides. In one embodiment the dimeric or multimeric FVIIa compound has an active site concentration which is at least 70%, preferably at least 80%, of the active site concentration of the constituent FVIIa polypeptides as measured by the assay disclosed herein (cf. Example 1). In another embodiment the dimeric or multimeric FVIIa compound has an increased biological activity as compared to the biological activity of the constituent FVIIa polypeptides.

In a preferred aspect of the invention, two FVIIa polypeptides are covalently connected to form a dimeric FVIIa compound. The resulting dimeric FVIIa compound thus has a molecular weight which is usually larger than approx. 100 kDa.

In another aspect the invention provides multimeric FVIIa compounds comprising at least three FVIIa polypeptides covalently connected such as to retain the intrinsic catalytic activity of the FVIIa polypeptides.

<u>FVIIa polypeptides</u>

A number of different FVIIa polypeptides may be covalently connected to form the dimeric or multimeric FVIIa compounds according to the present invention.

The term "Factor VII polypeptide" or "FVII polypeptide" as used herein means the inactive one-chain zymogen Factor VII molecule as well as variants thereof. The one chain zymogen Factor VII is a polypeptide comprising 406 amino acid residues, 10 of which are γ-carboxylated glutamic acid residues, N-glycosylated asparagines residues (no 145 and no 322), and O-glycosylated serine residues in position 52 and 60. The variant forms of FVII encompasses i.a. molecules wherein one or more amino acid residues have been substituted, added or deleted, molecules with different number of GLA residues, molecules with a modified or uncomplete glycosylation pattern. Non-limiting examples of modifications of amino acid residues are amidation, alkylation, acylation and PEGylation.

The term "Factor VIIa polypeptide" or "FVIIa polypeptide" as used herein means the active two-chain Factor VIIa molecule as well as variants thereof. The two-chain Factor VIIa is a polypeptide produced from FVII by hydrolysis of the $Arg_{152}$-$Ile_{153}$ peptide bond of FVII. FVIIa also comprises 406 amino acid residues, 10 of which are γ-carboxylated glutamic acid residues, N-glycosylated asparagines residues (no 145 and no 322), and O-glycosylated serine residues in position 52 and 60. The variant forms of FVIIa encompasses i.a. molecules wherein one or more amino acid residues have been substituted, added or deleted, molecules with different number of GLA residues, molecules with a modified or uncomplete glycosylation pattern.

The term "GLA" as used herein means 4-carboxyglutamic acid (γ-carboxyglutamate).

The term "polypeptide" as used herein means a compound comprising at least five constituent amino acid residues covalently connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxy-glutamic acid, ornithine, phophoserine, D-alanine, D-glutamic acid. Synthetic amino acids comprise amino acids manufactured by organic synthesis, e.g. D-isomers of the amino acids encoded by the genetic code and Aib (α-aminoisobutyric acid), Abu (α-aminobutyric acid), Tle (tert-butylglycine), and β-alanine. A polypeptide may comprise a single peptide chain or it may comprise more than one polypeptide chain, such as FVII being a single chain and FVIIa being two chains connected by disulphide bonds.

The term "Factor VIIa derivative" or "FVIIa derivative" as used herein, is intended to designate a FVIIa polypeptide exhibiting substantially the same or improved biological activity relative to wild-type Factor VIIa, in which one or more of the amino acids of the parent peptide have been genetically and/or chemically and/or enzymatically modified, e.g. by alkylation, glycosylation, deglycosylation, PEGylation, acylation, ester formation or amide formation or the like. This includes but is not limited to PEGylated Factor VIIa, cysteine-PEGylated human Factor VIIa and variants thereof.

The term "PEGylated Factor VIIa" (and the like) means a Factor VIIa polypeptide conjugated with a PEG molecule. It is to be understood, that the PEG molecule may be attached to any part of the Factor VIIa polypeptide including any amino acid residue or carbohydrate moiety of the Factor VIIa polypeptide. The term "cysteine-PEGylated Factor VIIa" means Factor VIIa polypeptide having a PEG molecule conjugated to a sulfhydryl group of a non-native cysteine of the Factor VIIa polypeptide.

Non-limiting examples of Factor VIIa derivatives includes GlycoPegylated FVIIa derivatives as disclosed in WO

03/31464 and US Patent applications US 20040043446, US 20040063911, US 20040142856, US 20040137557, and US 20040132640 (Neose Technologies, Inc.); FVIIa conjugates as disclosed in WO 01/04287, US patent application 20030165996, WO 01/58935, WO 03/93465 (Maxygen ApS) and WO 02/02764, US patent application 20030211094 (University of Minnesota).

The term "improved biological activity" refers to FVIIa polypeptides with i) substantially the same or increased proteolytic activity compared to recombinant wild type human Factor VIIa or ii) to FVIIa polypeptides with substantially the same or increased TF binding activity compared to recombinant wild type human Factor VIIa or iii) to FVIIa polypeptides with substantially the same or increased half life in blood plasma compared to recombinant wild type human Factor VIIa.

The term "PEGylated human Factor VIIa" means human Factor VIIa, having a PEG molecule conjugated to a human Factor VIIa polypeptide. It is to be understood, that the PEG molecule may be attached to any part of the Factor VIIa polypeptide including any amino acid residue or carbohydrate moiety of the Factor VIIa polypeptide. The term "cysteine-PEGylated human Factor VIIa" means Factor VIIa having a PEG molecule conjugated to a sulfhydryl group of a cysteine introduced in human Factor VIIa.

Non-limiting examples of Factor VIIa variants having substantially the same or increased proteolytic activity compared to recombinant wild type human Factor VIIa include S52A-FVIIa, S60A-FVIIa (Lino et al., Arch. Biochem. Biophys. 352: 182-192, 1998); FVIIa variants exhibiting increased proteolytic stability as disclosed in U.S. Patent No. 5,580,560; Factor VIIa that has been proteolytically cleaved between residues 290 and 291 or between residues 315 and 316 (Mollerup et al., Biotechnol. Bioeng. 48:501-505, 1995); oxidized forms of Factor VIIa (Kornfelt et al., Arch. Biochem. Biophys. 363:43-54, 1999); FVIIa variants as disclosed in PCT/DK02/00189 (corresponding to WO 02/077218); and FVIIa variants exhibiting increased proteolytic stability as disclosed in WO 02/38162 (Scripps Research Institute); FVIIa variants having a modified Gla-domain and exhibiting an enhanced membrane binding as disclosed in WO 99/20767, US patents US 6017882 and US 6747003, US patent application 20030100506 (University of Minnesota) and WO 00/66753, US patent applications US 20010018414, US 2004220106, and US 200131005, US patents US 6762286 and US 6693075 (University of Minnesota); and FVIIa variants as disclosed in WO 01/58935, US patent US 6806063, US patent application 20030096338 (Maxygen ApS), WO 03/93465 (Maxygen ApS), WO 04/029091 (Maxygen ApS), WO 04/083361 (Maxygen ApS), and WO 04/111242 (Maxygen ApS), as well as in WO 04/108763 (Canadian Blood Services).

Non-limiting examples of FVIIa variants having increased biological activity compared to wild-type FVIIa include FVIIa variants as disclosed in WO 01/83725, WO 02/22776, WO 02/077218, PCT/DK02/00635 (corresponding to WO 03/027147), Danish patent application PA 2002 01423 (corresponding to WO 04/029090), Danish patent application PA 2001 01627 (corresponding to WO 03/027147); WO 02/38162 (Scripps Research Institute); and FVIIa variants with enhanced activity as disclosed in JP 2001061479 (Chemo-Sero-Therapeutic Res Inst.).

Specific examples of interesting "engineered" Factors VIIa polypeptides are those disclosed in WO 02/077218 A1 (Novo Nordisk A/S) and WO 01/58935 A2 (Maxygen ApS) pages 21-24.

Particularly interesting are FVIIa polypeptides wherein a cysteine residue has been introduced. Examples of positions, wherein cysteine residues may be introduced include, but is not limited to, positions at or in the vicinity of the proteolytic degradation sites. Thus, in an interesting embodiment of the invention the cysteine residue(s) to be introduced, preferably by substitution, is selected from the group consisting of I30C, K32C, D33C, A34C, T37C, K38C, W41C, Y44C, S45C, D46C, L141C, E142C, K143C, R144C, L288C, D289C, R290C, G291C, A292C, S314C, R315C, K316C, V317C, L390C, M391C, R392C, S393C, E394C, P395C, R396C, P397C, G398C, V399C, L401C, R402C, A403C, P404C and combinations thereof, in particular selected from the group consisting of K32C, Y44C, K143C, R290C, R315C, K341C, R392C, R396C, R402C and combinations thereof. In a further interesting embodiment of the invention the cysteine residue(s) is/are introduced into a position that in wildtype hFVII is occupied by a threonine or serine residue having at least 25% of its side chain exposed to the surface. For instance, in the Factor VII polypeptide a cysteine residue is introduced, preferably by substitution, into at least one position selected from the group consisting of S12, S23, S43, S45, S52, S53, S60, S67, T83, S103, T106, T108, S111, S119, S126, T128, T130, S147, T185, S214, S222, S232, T233, T238, T239, T255, T267, T293, T307, S320, T324, S333, S336, T370 and S393. Even more preferable, the cysteine residue is introduced into at least one position of hFVII containing an S residue, the position being selected from the group consisting of S12, S23, S43, S45, S52, S53, S60, S67, S103, S111, S119, S126, S147, S214, S222, S232, S320, S333, S336 and S393. In a further embodiment the cysteine residue(s) is/are introduced into a position that in wildtype hFVII is occupied by a threonine or serine residue having at least 50% of its side chain exposed to the surface. For instance, in the Factor VII polypeptide a cysteine residue is introduced, preferably by substitution, into at least one position selected from the group consisting of S23, S43, S52, S53, S60, S67, T106, T108, S111, S119, S147, S214, T238, T267 and T293, even more preferable, a position selected from the group consisting of S23, S43, S52, S53, S60, S67, S111, S119, S147 and S214. In a still further embodiment a cysteine residue is introduced into at least one position selected from any of the above-mentioned positions, which is not located in an active site region. Preferably, the position is one occupied by a T or an S residue. As an example, the Factor VII polypeptide comprises a cysteine residue introduced into at least one position selected from the group consisting of S12, S23, S43, S45, S52, S53, S60,

S67, T83, S103, T106, T108, S111, S119, S126, T128, T130, S147, T185, S214, S222, T255, T267, T307, S320, S333, S336, T370 and S393 (having more than 25% of its side chain exposed to the surface), in particular selected from the group consisting of S12, S23, S43, S45, S52, S53, S60, S67, S103, S111, S1 19, S 126, S147, S214, S222, S320, S333, S336 and S393 (occupied by S residue), and, more preferable, from the group consisting of S23, S43, S52, S53, S60, S67, T106, T108, S111, S119, S147, S214 and T267 (having more than 50% of its side chain exposed to the surface), in particular from the group consisting of S23, S43, S52, S53, S60, S67, S111, S119, S147 and S214 (occupied by an S residue). In an even further embodiment a cysteine residue is introduced into at least one position selected from any of the above lists, which is not located in a tissue factor binding site region. Preferably, the position is one occupied by a T or an S residue. As an example, the Factor VII polypeptide comprises a cysteine residue introduced into at least one position selected from the group consisting of S12, S23, S45, S52, S53, S67, T83, S103, T106, T108, S111, S119, S126, T128, T130, S147, T185, S214, S222, S232, T233, T238, T239, T255, T267, T293, S320, T324, S333, S336, T370 and S393 (having more than 25% of its side chain exposed to the surface), in particular selected from the group consisting of S12, S23, S45, S52, S53, S67, S103, S111, S119, S126, S147, S214, S222, S232, S320, S333, S336 and S393 (occupied by S residue), and, more preferable, from the group consisting of S23, S52, S53, S67, T106, T108, S111, S119, S147, S214, T238, T267 and T293 (having more than 50% of its side chain exposed to the surface), in particular from the group consisting of S23, S52, S53, S67, S111, S119, S147 and S214 (occupied by an S residue). In a still further embodiment a cysteine residue is introduced into at least one position selected from any of the above lists, which is neither located in a tissue factor binding site region nor in an active site region. Preferably, the position is one occupied by a T or an S residue. As an example, the Factor VII polypeptide comprises a cysteine residue introduced into at least one position selected from the group consisting of S12, S23, S45, S52, S53, S67, T83, S103, T106, T108, S111, S119, S126, T128, T130, S147, T185, S214, S222, T255, T267, S320, S333, S336, T370 and S393 (having more than 25% of its side chain exposed to the surface), in particular selected from the group consisting of S12, S23, S45, S52, S53, S67, S103, S111, S119, S126, S147, S214, S222, S320, S333, S336 and S393 (occupied by S residue), and, more preferable, from the group consisting of S23, S52, S53, S67, T106, T108, S111, S119, S147, S214 and T267 (having more than 50% of its side chain exposed to the surface), in particular from the group consisting of S23, S52, S53, S67, S111, S119, S147 and S214 (occupied by an S residue).

Other useful examples of Factor VIIa polypeptide include those where an amino acid at a position selected from 247-260, 393-405 or 406, in particular R396, Q250 or P406, or K157, V158, M298, L305, D334, S336, K337 or F374 has been substituted with a cysteine, or where a cysteine has been introduced in the terminal, e.g. Factor VIIa 407C.

In a preferred series of embodiments the FVIIa polypeptide is FVIIa 407C, a FVIIa 407C variant, FVIIa P406C, a FVIIa P406C variant, FVIIa R396C, a FVIIa R396C variant, FVIIa Q250C or a FVIIa Q250C variant.

In another embodiment the FVIIa polypeptides are FVIIa variants having optimized GLA domains, e.g. combinations of Y4 (insertion), P10Q, K32E, D33F, D33E, and A34E.

In yet another embodiment the FVIIa polypeptides are FVIIa variants having increased intrinsic proteolytic activity, e.g. comprising substitutions selected from the group consisting of M298Q, V158D/E296V/M298Q, V158D/E296V/M298Q/K337A, F374Y/L305V, F374Y/L305V/S314E/K337A, F374Y/L305V/S314E, F374Y/L305V/K337A, L305V/K337A, V158D/E296V/M298Q/L305V, V158D/E296V/M298Q/L305V/K337A, V158T/M298Q, E296V/M298Q, V158D/E296V, V158D/M298Q and the 407C variants thereof.

In yet another embodiment the FVIIa polypeptide is a FVIIa variant comprising substitutions selected from the group consisting of L305V, L305V/M306D/D309S, L305I, L305T, F374P, V158T/M298Q, V158D/E296V/M298Q, K337A, M298Q, V158D/M298Q, L305V/K337A, V158D/E296V/M298Q/L305V, V158D/E296V/M298Q/K337A, V158D/E296V/M298Q/L305V/K337A, K157A, E296V, E296V/M298Q, V158D/E296V, V158D/M298K, S336G, L305V/K337A, L305V/V158D, L305V/E296V, L305V/M298Q, L305V/V158T, L305V/K337A/V158T, L305V/K337A/M298Q, L305V/K337A/E296V, L305V/K337A/V158D, L305V/V158D/M298Q, L305V/V158D/E296V, L305V/V158T/M298Q, L305V/V158T/E296V, L305V/E296V/M298Q, L305V/V158D/E296V/M298Q, L305V/V158T/E296V/M298Q, L305V/V158T/K337A/M298Q, L305V/V158T/E296V/K337A, L305V/V158D/K337A/M298Q, L305V/V158D/E296V/K337A, L305V/V158D/E296V/M298Q/K337A, L305V/V158T/E296V/M298Q/K337A, S314E/K316H, S314E/K316Q, S314E/L305V, S314E/K337A, S314E/V158D, S314E/E296V, S314E/M298Q, S314E/V158T, K316H/L305V, K316H/K337A, K316H/V158D, K316H/E296V, K316H/M298Q, K316H/V158T, K316Q/L305V, K316Q/K337A, K316Q/VI58D, K316Q/E296V, K316Q/M29SQ, K316Q/V158T, S314E/L305V/K337A, S314E/L305V/V158D, S314E/L305V/E296V, S314E/L305V/M298Q, S314E/L305V/V158T, S314E/L305V/K337A/V158T, S314E/L305V/K337A/M298Q, S314E/L305V/K337A/E296V, S314E/L305V/K337A/V158D, S314E/L305V/V158D/M298Q, S314E/L305V/V158D/E296V, S314E/L305V/V158T/M298Q, S314E/L305V/V158T/E296V, S314E/L305V/E296V/M298Q, S314E/L305V/V158D/E296V/M298Q, S314E/L305V/V158T/E296V/M298Q, S314E/L305V/V158T/K337A/M298Q, S314E/L305V/V158T/E296V/K337A, S314E/L305V/V158D/K337A/M298Q, S314E/L305V/V158D/E296V/K337A, S314E/L305V/VI58D/E296V/M298Q/K337A, S314E/L305V/VI58T/E296V/M298Q/K337A, K316H/L305V/K337A, K316H/L305V/V158D, K316H/L305V/E296V, K316H/L305V/M298Q, K316H/L305V/V158T,

K316H/L305V/K337A/V158T, K316H/L305V/K337A/M298Q, K316H/L305V/K337A/E296V, K316H/L305V/K337A/V158D, K316H/L305V/V158D/M298Q, K316H/L305V/V158D/E296V, K316H/L305V/V158T/M298Q, K316H/L305V/V158T/E296V, K316H/L305V/E296V/M298Q, K316H/L305V/V158D/E296V/M298Q, K316H/L305V/V158T/E296V/M298Q, K316H/L305V/V158T/K337A/M298Q, K316H/L305V/V158T/E296V/K337A, K316H/L305V/V158D/K337A/M298Q, K316H/L305V/V158D/E296V/K337A, K316H/L305V/V158D/E296V/M298Q/K337A, K316H/L305V/V158T/E296V/M298Q/K337A, K316Q/L305V/K337A, K316Q/L305V/Vl58D, K316Q/L305V/E296V, K316Q/L305V/M298Q, K316Q/L305V/V158T, K316Q/L305V/K337A/V158T, K316Q/L305V/K337A/M298Q, K316Q/L305V/K337A/E296V, K316Q/L305V/K337A/V158D, K316Q/L305V/Vl5SD/M298Q, K316Q/L305V/V158D/E296V, K316Q/L305V/V158T/M298Q, K316Q/L305V/V158T/E296V, K316Q/L305V/E296V/M298Q, K316Q/L305V/V158D/E296V/M298Q, K316Q/L305V/V158T/E296V/M298Q, K316Q/L305V/V158T/K337A/M298Q, K316Q/L305V/V158T/E296V/K337A, K316Q/L305V/V158D/K337A/M298Q, K316Q/L305V/Vl58D/E296V/K337A, K316Q/L305V/V158D/E296V/M298Q/K337A, K316Q/L305V/V158T/E296V/M298Q/K337A, and the 407C variants thereof.

In yet another embodiment the FVIIa polypeptides are PEGylated. It is to be understood that the PEGylation can be performed prior to the formation of the dimeric or multimeric FVIIa compound, or it can be performed after the formation of the dimeric or multimeric FVIIa compound.

In yet another embodiment the FVIIa compound is a dimer containing two FVIIa polypeptides. In yet another embodiment the FVIIa compound is a trimer containing three FVIIa polypeptides. In yet another embodiment the FVIIa compound is a tetramer containing four FVIIa polypeptides.

Dimeric and multimeric FVIIa compounds

A range of coupling chemistries may be used for producing the dimeric or multimeric FVIIa compounds which may optionally encompass a linker moiety.

In a preferred embodiment the FVIIa polypeptides covalently connected to form the dimeric or multimeric FVIIa compounds are identical FVIIa polypeptides. In another embodiment the FVIIa polypeptides covalently connected to form the dimeric or multimeric FVIIa compounds are two different FVIIa polypeptides.

In a particularly preferred embodiment the dimeric or multimeric FVIIa compounds comprise at least two FVIIa polypeptides which are covalently connected via engineered cysteines in said FVIIa polypeptides. For the synthesis of such dimeric or multimeric FVIIa compounds comprising FVIIa polypeptides being connected via cysteines, the method disclosed below for producing selectively reduced FVIIa polypeptides is useful. In another embodiment the dimeric or multimeric FVIIa compounds according to the invention comprise a linker between the constituent FVIIa polypeptides. One class of such linkers is linkers having the structure that results from a reaction involving a bivalent cysteine reactive PEG. One such preferred linker has the structure that results from a reaction involving maleimide-PEG-maleimide, e.g. maleimide-PEG2kD-maleimide, maleimide-PEG3.4kD-maleimide, maleimide-PEG5kD-maleimide, maleimide-PEG10kD-maleimide, and maleimide-PEG20kD-maleimide. In another embodiment said linker comprises an amino acid sequence.

In yet another embodiment the dimeric or multimeric FVIIa compounds according to the invention comprises FVIIa polypeptides that are covalently connected via free amines.

In yet another embodiment the dimeric or multimeric FVIIa compounds according to the invention comprises FVIIa polypeptides that are covalently connected via natural or engineered glycans attached to said FVIIa polypeptides.

In yet another embodiment the dimeric or multimeric FVIIa compounds according to the invention comprises at least two FVIIa polypeptides that are covalently connected via a chemical structure resulting from an enzyme catalyzed coupling reaction, e.g. by transglutaminase or sortase.

In the situation where the dimeric or multimeric FVIIa compounds comprise FVIIa polypeptides covalently connected via engineered cysteines, preferred FVIIa polypeptides are selected from FVIIa 407C, a FVIIa 407C variant, FVIIa P406C, a FVIIa P406C variant, FVIIa R396C, a FVIIa R396C variant, FVIIa Q250C and a FVIIa Q250C variant.

Also within the scope of the present invention is conjugates between the dimeric or multimeric FVIIa compounds and a protractor group, such as human serum albumin or a variant thereof, a PEG moiety, a lipophilic moiety etc.

In general it is preferred to prepare a dimeric or multimeric FVIIa compound according to the invention by a method comprising the steps :

a) synthesis and purification of said FVIIa polypeptides;
b) synthesis of said compound by coupling said FVIIa polypeptides;
c) isolation of said dimeric or multimeric FVIIa compound.

When employing cysteine coupling chemistry it is preferred to prepare a dimeric or multimeric FVIIa compound

according to the invention by a method comprising the steps :

a) synthesis and purification of said FVIIa polypeptides;
b) selective reduction of the cysteine residue on the FVIIa polypeptides which are to be linked;
c) synthesis of said dimeric or multimeric FVIIa compound by coupling said FVIIa polypeptides with reduced cysteine residues in the presence of an activated linker;
d) isolation of said dimeric or multimeric FVIIa compound.

In a preferred embodiment of the method, step b) comprises selective reduction of the cysteine residue on the FVIIa polypeptide by reaction with a redox buffer or a triarylphosphine reducing agent (cf section below on selective reduction).

Selective reduction of non-native cysteine of FVIIa monomer.

As mentioned above, when the dimeric or multimeric FVIIa compound is to be synthesized from the constituent FVIIa molecules or FVII molecules via non-native cysteines it may be necessary to selectively reduce the non-native cysteines in the FVIIa or FVII polypeptides prior to coupling. The FVIIa and FVII polypeptides typically comprises one or more cysteine moieties conjugated through a disulfide bridge to a low-molecular weight thiol (RS-Cys), said moiety/moieties not being involved in intramolecular S-S bridges (Cys-S-S-Cys) when the FVIIa polypeptide is in its active form.

Selective reduction using redox buffer.

One method for the selective reduction comprises the step of allowing the low-molecular weight thiol-conjugated FVIIa polypeptide to react with a mixture comprising a redox buffer.

When used herein, the term "redox buffer" is intended to mean a thiol/disulfide redox pair in a ratio that is sufficiently reducing to disrupt the FVIIa or FVII polypeptide-low-molecular weight thiol mixed disulfide(s) (RS-Cys) and at the same time sufficiently oxidizing to preserve the integrity of the native disulfide bonds in the FVIIa polypeptide.

Preferably, the redox buffer comprises a low molecular weight thiol/disulfide redox pair. By the term "low molecular weight" is meant that the thiol-form of the redox pair has a molecular weight of at the most 500 g/mol. Illustrative examples of such redox pairs are the ones selected from (i) reduced and oxidized glutathione and (ii) reduced and oxidized y-glutamylcysteine, (iii) reduced and oxidized cysteinylglycine, (iv) reduced and oxidized cysteine, (v) reduced and oxidized N-acetylcysteine, (vi) cysteamine, and (vii) dihydrolipoamide/lipoamide, preferably from (i) reduced and oxidized glutathione.

The optimal redox conditions can be determined by performing a redox titration of the protein as known to the person skilled in the art and as demonstrated in Figures 2, 3, and 4; see also Gilbert (1995).

In one embodiment, the redox buffer is a redox pair of reduced and oxidized glutathione, and the concentration of the reduced glutathione is in the range of 0-100 mM, and the ratio between reduced glutathione and oxidized glutathione is in the range of 2-200.

In another embodiment, the redox buffer is a redox pair of reduced and oxidized glutathione, and the concentration of the reduced glutathione is in the range of 0-100 mM, e.g. 0.01-50 mM, and the concentration of the oxidized glutathione is in the range of 0-5 mM, e.g. 0.001-5 mM. For Factor VII polypeptides, the concentration of the reduced glutathione is preferably in the range of 0-5 mM, e.g. 0.01-2 mM, and the concentration of the oxidized glutathione is in the range of 0.001-2 mM, e.g. 0.001-0.200 mM.

Since glutathione and other low molecular-weight thiols are generally poor reductants/-oxidants in terms of reaction kinetics, a thiol/disulfide redox catalyst is most preferably included in the mixture in conjunction with the redox buffer in order to enhance the rate of the reaction.

Suitable thiol/disulfide redox catalysts to be included in the mixture include dithiol-type and monothiol-type glutaredoxins. Glutaredoxins and their functions are generally described in Fernandes et al. (2004). Useful examples of glutaredoxins are those selected from Grx1, Grx2 or Grx3 from *Escherichia coli* (Holmgren et al., 1995), Grx1p, Grx2p, Grx3p, Grx4p, and Grx5p from *Saccharomyces cerevisiae* (Luikenhuis et al. 1998; Rodriguez-Manzaneque et al., 1999; Grant, 2001), Grx1 and Grx2 from *Homo sapiens* (Padilla et al. 1995; Lundberg et al., 2001), and variants hereof. Variants include, but is not restricted to, dithiol-type glutaredoxins in which the C-terminal cysteine in the CXXC motif has been replaced by another amino acid typically serine (see Yang et al., 1998).

The redox catalyst (in particular a glutaredoxin) is preferably used in a concentration of 0.001-20 $\mu$M.

It is preferred that the mixture does not comprise a protein disulfide isomerase (PDI).

The redox buffer may further comprise other components such as salts, pH buffers, etc., and the method of the invention may be conducted at any temperature which is suitable for the FVIIa protein in question, e.g. a temperature in the range of from -5°C to 50°C, such as in the range of from 0°C to 25°C, of course dependent on the stability of the

protein under the given conditions.

Selective reduction using a triarylphosphine reducing agent.

Another method for the selective reduction comprises the step of allowing the low-molecular weight thiol-conjugated FVIIa protein to react with a triarylphosphine reducing agent.

The term "triarylphosphine reducing agent" is intended to mean a triarylphosphine optionally substituted with one or more substituents.

The aryl groups of the triarylphosphine reducing agent are preferably selected from phenyl, naphthyl, 1,2,3,4-tetrahydronaphthyl, anthracyl, phenanthracyl, pyrenyl, benzopyrenyl, fluorenyl and xanthenyl, in particular phenyl, and in currently selected embodiments, the aryl groups are preferably identical. In the currently most interesting embodiment, all three aryl groups are phenyl. Examples of substituents, which may be present in the aryl groups, in particular phenyl groups, are typically those selected from sulfonic acid, carboxylic acid, $C_{1-6}$-alkyl, $C_{1-6}$-alkoxy, and $C_{1-6}$-alkoxy-$C_{1-6}$-alkyl, or $C_{3-6}$-alkylene (representing a ring with two neighboring aryl carbon atoms) or $C_{2-6}$-alkyleneoxy (representing a ring with two neighboring aryl carbon atoms) or $C_{1-4}$-alkylene-oxy-$C_{1-4}$-alkylen (representing a ring with two neighboring aryl carbon atoms).

In one embodiment, at least one aryl (e.g. phenyl) has at least one substituent selected from sulfonic acid and carboxylic acid, in particular sulfonic acid; such substituent preferably being arranged in the meta position relative to the bond to the phosphor atom.

Preferably, all three aryl groups have a sulfonic acid substituent, e.g. all three aryl groups have a sulfonic acid substituent and at least one further substituent, in particular at least a substituent in the para-position relative to the bond to the phosphor atom, in particular an oxygen substituent in this para-position.

It is currently believed that the aryl groups of preferred reducing agents do not have any substituents in the ortho-position relative to the bond to the phosphor atom.

The term "$C_{1-6}$-alkyl" is intended to encompass linear or branched saturated hydrocarbon residues which have 1-6 carbon atoms. Particular examples are methyl, ethyl, n-propyl, isopropyl, n-butyl, isobutyl, sec-butyl, tert-butyl, n-pentyl, isopentyl, n-hexyl, etc. Similarly, the term "$C_{1-4}$-alkyl." encompasses linear or branched saturated hydrocarbon residues which have 1-4 carbon atoms. The terms "$C_{1-6}$-alkylene", "$C_{2-6}$-alkylene", etc. represent the biradicals corresponding to "$C_{1-6}$-alkyl", "$C_{2-6}$-alkyl", respectively.

Suitable triarylphosphine reducing agents are those having a useful balance between reduction potential and steric hinderance. The chemical nature of the triarylphosphine reducing agent is dictated by its ability to cleave the protein-low-molecular weight thiol mixed disulfide (RS-Cys) while preserving the integrity of the native disulfide bonds in the protein. Currently very interesting compounds are triarylphosphine trisulfonic acids, such as triphenylphosphine-3,3',3"-trisulfonic acid and analogues hereof. Illustrative examples hereof are triarylphosphine reducing agents selected from triphenylphosphine-3,3',3"-trisulfonic acid and analogues thereof, e.g. one of those selected from the compounds 9-11 below:

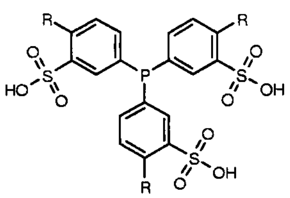

R = H, Me, Et, Pr, 2-Pr, Bu, MeO, EtO, PrO, 2-PrO, BuO.

**9**

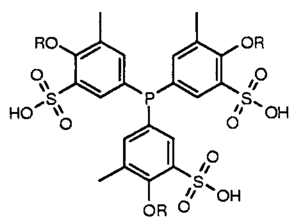

R = Me, Et, Pr, 2-Pr, Bu.

**10**

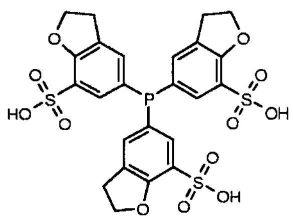

**11**

The triarylphosphine reducing agent is preferably used in a concentration of 0.001-100 mM, such as 0.01-50 mM or 0.1-25 mM.

In one interesting embodiment, the triarylphosphine reducing agent is immobilized to a solid support. This will facilitate the easy separation of the reducing agent from the protein. In general, triarylphosphine reducing agent, such as compounds **9-11,** may be immobilized by means known to the person skilled in the art, e.g. by introducing a linker group in one of the aryl groups. The triarylphosphine reagent **12** is an example of a linkable variant of **1**.

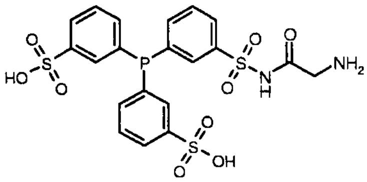

12

The reaction is typically conducted at a temperature in the range of -40°C, such as at ambient temperature, for a period of from 5 seconds to several days, as the case may be. The reaction may be followed by HPLC in order to confirm the conversion. The solvent is preferably an aqueous buffer, optionally including co-solvents such as DMSO or DMF. The solvent may also comprise salts, e.g. calcium salts.

In one variant, the triarylphosphine reducing agent is used in combination with an inhibitor for the protein, e.g. an active site $S_1$ pocket inhibitor, see further below.

In one currently preferred embodiment, the method for selective reduction of a FVIIa or FVII polypeptide comprising one or more cysteine moieties conjugated through a disulfide bridge to a low-molecular weight thiol (RS-Cys), said moiety/moieties not being involved in intramolecular S-S bridges (Cys-S-S-Cys) when the FVIIa polypeptide is in its active form, the method comprising the step of allowing the low-molecular weight thiol-conjugated FVIIa or FVII polypeptide to react with a mixture comprising reduced and oxidized glutathione and a glutaredoxin, and the simultaneous and/or subsequent step of conjugating at least one of the selectively reduced cysteine (HS-Cys) moieties with a chemical group.

In another currently preferred embodiment, the method for selective reduction of a FVIIa or FVII polypeptide comprising one or more cysteine moieties conjugated through a disulfide bridge to a low-molecular weight thiol (RS-Cys), said moiety/moieties not being involved in intramolecular S-S bridges(Cys-S-S-Cys) when the FVIIa polypeptide is in its active form, the method comprising the step of allowing the low-molecular weight thiol-conjugated FVIIa polypeptide to react with a mixture comprising a triarylphosphine-3,3',3"-trisulfonic acid compound and an active site $S_1$ pocket inhibitor, and the simultaneous and/or subsequent step of conjugating at least one of the selectively reduced cysteine (HS-Cys) moieties with a chemical group.

The FVIIa polypeptide

The selective reduction strategy using a redox buffer as described above is believed to be generally applicable for engineered FVIIa or FVII polypeptides comprising at least one non-native cysteine, in particular such proteins having engineered cysteines not being involved in intramolecular S-S bridges (Cys-S-S-Cys) when the protein is in its active form, and therefore potentially being in low-molecular weight thiol-conjugated (RS-Cys) form.

The term "low-molecular weight thiol-conjugated (RS-Cys) form" and similar terms are intended to mean that a thiol group of a cysteine of the FVIIa polypeptide in question is conjugated with a compound having a thiol group, wherein said compound has a molecular weight of less than 500 Da. Examples of such compounds are glutathione, gamma-glutamylcysteine, cysteinylglycine, cysteine, homocysteine, N-acetylcysteine, cysteamine, etc.

The term "active form" refers to the form (or forms) of the protein wherein it is capable of performing a desirable action, e.g. as a catalyst (enzyme), zymogen, or as a co-factor, etc. The "active form" is sometimes referred to as the "correctly folded form".

In one interesting embodiment, a substantial portion of the FVIIa polypeptide (i.e. at least 50%) is in its active form when the selective reduction reaction is conducted.

The FVIIa polypeptide is generally an "engineered" polypeptide which compared to native FVIIa includes at least one non-native cysteine. Such "engineered" polypeptides are preferably prepared by recombinant techniques as will be apparent for the person skilled in the art; see also WO 02/077218 A1 and WO 01/58935 A2.

Protein inhibitor.

In one interesting embodiment, the mixture further comprises an inhibitor of the FVIIa polypeptide. By including an inhibitor in the mixture, it is believed that the conformation of the protein is somewhat stabilized whereby the intramolecular disulfide bonds have a lower tendency to become reduced by the redox buffer. Preferably, the inhibitor of the protein is an active-site inhibitor.

In the case of a FVIIa polypeptide, the presence of an active-site inhibitor extending into the $S_1$ binding pocket might be required during the selective reduction reaction to protect internal disulfide bonds in the active-site region from reduction. Inhibitors useful for this purpose include benzamidines, such as 4-aminobenzamidine, arginines, and other

more potent analogues, see, e.g., those disclosed in WO 05/016365 A3 and those disclosed by Aventis in EP 1 162 194 A1, cf. in particular those defined in claims 1-6 and in sections [0009]-[0052], and in EP 1 270 551 A1, cf. in particular claims 1 and 2 and sections [0010]-[0032].

Conjugates

One important purpose of the selective reduction methods described above is to liberate a cysteine group which can be used for attachment (conjugation) of a chemical group, e.g. a non-polypeptide moiety.

Hence, in one important embodiment, the method further comprises the simultaneous and/or subsequent step of conjugating at least one of the selectively reduced cysteine (HS-Cys) moiety/moieties with a chemical group.

It should be understood that the conjugation of the at least one selectively reduced cysteine moieties with a chemical group may be conducted simultaneously, i.e. by addition of one or more reagents leading to the conjugation to the mixture comprising the redox buffer, or in a subsequent step, e.g. after purification and/or isolation of the selectively reduced protein.

It is also to be understood that conjugation may be conducted on a FVIIa polypeptide before synthesis of the dimeric or polymeric FVIIa compound, or it may be conducted after the dimeric or polymeric FVIIa compound has been synthesized.

In one embodiment, the chemical group is a protractor group, i.e. a group which upon conjugation to the FVIIa polypeptide increases the circulation half-life of said FVIIa polypeptide, when compared to the un-modified polypeptide. The specific principle behind the protractive effect may be caused by increased size, shielding of peptide sequences that can be recognized by peptidases or antibodies, or masking of glycanes in such way that they are not recognized by glycan specific receptores present in e.g. the liver or on macrophages, preventing or decreasing clearance. The protractive effect of the protractor group can e.g. also be caused by binding to blood components sush as albumin, or unspecific adhesion to vascular tissue. The conjugated glycoprotein should substantially preserve its biological activity.

In one embodiment of the invention the protractor group is selected from the group consisting of:

(a) A low molecular organic charged radical (15-1,000 Da), which may contain one or more carboxylic acids, amines, sulfonic acids, tetrazoles, acylsulfonamides, phosphonic acids, or combination thereof.

(b) A low molecular (15-1,000 Da) neutral hydrophilic molecule, such as cyclodextrin, or a polyethylene chain which may optionally branched.

(c) A low molecular (15-1,000 Da) hydrophobic molecule such as a fatty acid or cholic acid or derivatives thereof.

(d) Polyethyleneglycol with an average molecular weight of 2,000-60,000 Da.

(e) A well defined precision polymer such as a dendrimer with an exact molecular mass ranging from 700 to 20,000 Da, or more preferable between 700-10,000 Da.

(f) A substantially non-immunogenic polypeptide such as albumin or an antibody or part of an antibody optionally containing an Fc-domain.

(g) A high molecular weight organic polymer such as dextran.

In another embodiment of the invention the protractor group is selected from the group consisting of dendrimers, polyalkylene oxide (PAO), including polyalkylene glycol (PAG), such as polyethylene glycol (PEG) and polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride, and dextran, including carboxymethyl-dextran. In one particularly interesting embodiment of the invention, the protractor group is a PEG group.

The term "branched polymer", or interchangebly "dendritic polymer", "dendrimer" or "dendritic structure" means an organic polymer assembled from a selection of monomer building blocks of which, some contains branches.

In one embodiment of the invention the protractor group is a selected from the goup consisting of serum protein binding-ligands, such as compounds which bind to albumin, like fatty acids, C5-C24 fatty acid, aliphatic diacid (e.g. C5-C24). Other examples of protractor groups includes small organic molecules containing moieties that under physiological conditions alters charge properties, such as carboxylic acids or amines, or neutral substituents that prevent glycan specific recognition such as smaller alkyl substituents (e.g., C1-C5 alkyl). In one embodiment of the invention the protractor group is albumin.

In one embodiment, the chemical group is a non-polypeptide.

In one interesting embodiment, the chemical group is a polyethyleneglycol (PEG), in particular one having an average molecular weight of in the range of 500-100,000, such as 1,000-75,000, or 2,000-60,000.

Conjugation can be conducted as disclosed in WO 02/077218 A1 and WO 01/58935 A2.

Particularly interesting is the use of PEG as a chemical group for conjugation with the protein. The term "polyethylene glycol" or "PEG" means a polyethylene glycol compound or a derivative thereof, with or without coupling agents, coupling or activating moeities (e.g., with thiol, triflate, tresylate, azirdine, oxirane, pyridyldithio, vinyl sulfone, or preferably with a maleimide moiety). Compounds such as maleimido monomethoxy PEG are exemplary of activated PEG compounds of the invention.

PEG is a suitable polymer molecule, since it has only few reactive groups capable of cross-linking compared to polysaccharides such as dextran. In particular, monofunctional PEG, e.g. methoxypolyethylene glycol (mPEG), is of interest since its coupling chemistry is relatively simple (only one reactive group is available for conjugating with attachment groups on the FVIIa polypeptide). Consequently, the risk of cross-linking is eliminated, the resulting FVIIa polypeptide conjugates are more homogeneous and the reaction of the polymer molecules with the FVIIa polypeptide is easier to control.

To effect covalent attachment of the polymer molecule(s) to the FVIIa polypeptide, the hydroxyl end groups of the polymer molecule are provided in activated form, i.e. with reactive functional groups. Suitable activated polymer molecules are commercially available, e.g. from Shearwater Corp., Huntsville, Ala., USA, or from PolyMASC Pharmaceuticals plc, UK. Alternatively, the polymer molecules can be activated by conventional methods known in the art, e.g. as disclosed in WO 90/13540. Specific examples of activated linear or branched polymer molecules for use in the present invention are described in the Shearwater Corp. 1997 and 2000 Catalogs (Functionalized Biocompatible Polymers for Research and pharmaceuticals, Polyethylene Glycol and Derivatives, incorporated herein by reference). Specific examples of activated PEG polymers include the following linear PEGs: NHS-PEG (e.g. SPA-PEG, SSPA-PEG, SBA-PEG, SS-PEG, SSA-PEG, SC-PEG, SG-PEG, and SCM-PEG), and NOR-PEG), BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in U.S. Pat. No. 5,932,462 and U.S. Pat. No. 5,643,575, both of which are incorporated herein by reference. Furthermore, the following publications, incorporated herein by reference, disclose useful polymer molecules and/or PEGylation chemistries: U.S. Pat. No. 5,824,778, U.S. Pat. No. 5,476,653, WO 97/32607, EP 229,108, EP 402,378, U.S. Pat. No. 4,902,502, U.S. Pat. No. 5,281,698, U.S. Pat. No. 5,122,614, U.S. Pat. No. 5,219,564, WO 92/16555, WO 94/04193, WO 94/14758, WO 94/17039, WO 94/18247, WO 94/28024, WO 95/00162, WO 95/11924, WO 95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 439 508, WO 97/03106, WO 96/21469, WO 95/13312, EP 921 131, U.S. Pat. No. 5,736,625, WO 98/05363, EP 809 996, U.S. Pat. No. 5,629,384, WO 96/41813, WO 96/07670, U.S. Pat. No. 5,473,034, U.S. Pat. No. 5,516,673, EP 605 963, U.S. Pat. No. 5,382,657, EP 510 356, EP 400 472, EP 183 503 and EP 154 316.

The conjugation of the FVIIa polypeptide and the activated polymer molecules is conducted by use of any conventional method, e.g. as described in the following references (which also describe suitable methods for activation of polymer molecules): R. F. Taylor, (1991), "Protein immobilisation. Fundamental and applications", Marcel Dekker, N.Y.; S. S. Wong, (1992), "Chemistry of Protein Conjugation and Crosslinking", CRC Press, Boca Raton; G. T. Hermanson et al., (1993), "Immobilized Affinity Ligand Techniques", Academic Press, N.Y.). The skilled person will be aware that the activation method and/or conjugation chemistry to be used depends on the attachment group(s) of the polypeptide (examples of which are given further above), as well as the functional groups of the polymer (e.g. being amine, hydroxyl, carboxyl, aldehyde, sulfydryl, succinimidyl, maleimide, vinysulfone or haloacetate). The PEGylation may be directed towards conjugation to all available attachment groups on the FVIIa polypeptide (i.e. such attachment groups that are exposed at the surface of the FVIIa polypeptide) or may be directed towards one or more specific attachment groups, e.g. the N-terminal amino group (U.S. Pat. No. 5,985,265). Furthermore, the conjugation may be achieved in one step or in a stepwise manner (e.g. as described in WO 99/55377).

It will be understood that the PEGylation is designed so as to produce the optimal molecule with respect to the number of PEG molecules attached, the size and form of such molecules (e.g. whether they are linear or branched), and where in the polypeptide such molecules are attached. The molecular weight of the polymer to be used will be chosen taking into consideration the desired effect to be achieved. For instance, if the primary purpose of the conjugation is to achieve a conjugate having a high molecular weight and larger size (e.g. to reduce renal clearance), one may choose to conjugate either one or a few high molecular weight polymer molecules or a number of polymer molecules with a smaller molecular weight to obtain the desired effect. Preferably, however, several polymer molecules with a lower molecular weight will be used.

It has further been found that advantageous results are obtained when the apparent size (also referred to as the "apparent molecular weight" or "apparent mass") of at least a major portion of the conjugate of the invention is at least about 50 kDa, such as at least about 55 kDa, such as at least about 60 kDa, e.g. at least about 66 kDa. This is believed to be due to the fact that renal clearance is substantially eliminated for conjugates having a sufficiently large

apparent size. In the present context, the "apparent size" of a FVIIa polypeptide is determined by the SDS-PAGE method.

Furthermore, it has been reported that excessive polymer conjugation can lead to a loss of activity of the FVIIa polypeptide to which the chemical group (e.g. a non-polypeptide moiety) is conjugated (see further below). This problem can be eliminated, e.g., by removal of attachment groups located at the functional site or by reversible blocking the functional site prior to conjugation so that the functional site of the FVIIa polypeptide is blocked during conjugation. Specifically, the conjugation between the FVIIa polypeptide and the chemical group (e.g. non-polypeptide moiety) may be conducted under conditions where the functional site of the FVIIa polypeptide is blocked by a helper molecule e.g. tissue factor capable of binding to the functional site of the FVIIa polypeptide or a serine protease inhibitor. Preferably, the helper molecule is one, which specifically recognizes a functional site of the FVIIa polypeptide, such as a receptor, in particular tissue factor, either full length or a suitably truncated form of tissue factor or two molecules, one being tissue factor the other one being a peptide or peptide inhibitor binding to and thus protecting the area around the catalytic triad (preferably defined as amino acid residues within 10 Å of any atom in the catalytic triad).

Alternatively, the helper molecule may be an antibody, in particular a monoclonal antibody recognizing the FVIIa polypeptide. In particular, the helper molecule may be a neutralizing monoclonal antibody.

The FVIIa polypeptide is preferably to interact with the helper molecule before effecting conjugation. (Often it is even advantageous to use the same helper molecule (e.g. an inhibitor) as the one used in the steps where mixed disulfides are reduced.) This ensures that the functional site of the FVIIs polypeptide is shielded or protected and consequently unavailable for derivatization by the chemical group (e.g. non-polypeptide moiety) such, as a polymer.

Following its elution from the helper molecule, the conjugate of the chemical group and the protein can be recovered with at least a partially preserved functional site.

Pharmaceutical compositions.

The dimeric and multimeric FVIIa compounds according to the present invention are applicable as pharmaceutical compositions for the treatment of bleeding disorders or bleeding episodes in a subject or for the enhancement of the normal haemostatic system. Examples of subjects in need of such treatment are i.a. subjects being treated for haemophilia A or B.

In another aspect, the present invention includes within its scope pharmaceutical compositions comprising a dimeric or multimeric FVIIa compound, as an active ingredient, or a pharmaceutically acceptable salt thereof together with a pharmaceutically acceptable carrier or diluent.

Optionally, the pharmaceutical composition of the invention may comprise a dimeric or multimeric FVIIa compound in combination with one or more other compounds exhibiting anticoagulant activity, e.g., platelet aggregation inhibitor.

The compounds of the invention may be formulated into pharmaceutical composition comprising the compounds and a pharmaceutically acceptable carrier or diluent. Such carriers include water, physiological saline, ethanol, polyols, e.g., glycerol or propylene glycol, or vegetable oils. As used herein, "pharmaceutically acceptable carriers" also encompasses any and all solvents, dispersion media, coatings, antifungal agents, preservatives, isotonic agents and the like. Except insofar as any conventional medium is incompatible with the active ingredient and its intended use, its use in the compositions of the present invention is contemplated.

The compositions may be prepared by conventional techniques and appear in conventional forms, for example, capsules, tablets, solutions or suspensions. The pharmaceutical carrier employed may be a conventional solid or liquid carrier. Examples of solid carriers are lactose, terra alba, sucrose, talc, gelatine, agar, pectin, acacia, magnesium stearate and stearic acid. Examples of liquid carriers are syrup, peanut oil, olive oil and water. Similarly, the carrier or diluent may include any time delay material known to the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilised and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound to the appropriate or desired site of action, such as oral or parenteral, e.g., rectal, transdermal, subcutaneous, intranasal, intramuscular, topical, intravenous, intraurethral, ophthalmic solution or an ointment, the oral route being preferred.

If a solid carrier for oral administration is used, the preparation can be tabletted, placed in a hard gelatine capsule in powder or pellet form or it can be in the form of a troche or lozenge. The amount of solid carrier may vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension

or solution.

For nasal administration, the preparation may contain a compound of formula (I) dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet, which may be prepared by conventional tabletting techniques, contains

| | |
|---|---|
| Core: | |
| Active compound (as free compound or salt thereof) | 10 mg |
| Colloidal silicon dioxide (Areosil®) | 1.5 mg |
| Cellulose, microcryst. (Avicel®) | 70 mg |
| Modified cellulose gum (Ac-Di-Sol®) | 7.5 mg |
| Magnesium stearate | |

| | |
|---|---|
| Coating: | |
| HPMC | approx. 9 mg |
| *Mywacett® 9-40 T | approx. 0.9 mg |
| *Acylated monoglyceride used as plasticizer for film coating. | |

The compounds of the invention may be administered to a mammal, especially a human in need of such treatment, prevention, elimination, alleviation or amelioration of various thrombolytic or coagulophatic diseases or disorders as mentioned above. Such mammals also include animals, both domestic animals, e.g. household pets, and non-domestic animals such as wildlife.

Usually, dosage forms suitable for oral, nasal, pulmonal or transdermal administration comprise from about 0.001 mg to about 100 mg, preferably from about 0.01 mg to about 50 mg of the compounds of formula I admixed with a pharmaceutically acceptable carrier or diluent.

The compounds may be administered concurrently, simultaneously, or together with a pharmaceutically acceptable carrier or diluent, whether by oral, rectal, or parenteral (including subcutaneous) route. The compounds are often, and preferably, in the form of an alkali metal or earth alkali metal salt thereof.

Suitable dosage ranges varies as indicated above depending upon the exact mode of administration, form in which administered, the indication towards which the administration is directed, the subject involved and the body weight of the subject involved, and the preference and experience of the physician or veterinarian in charge.

The present invention is further illustrated by the following examples which, however, are not to be construed as limiting the scope of protection. The features disclosed in the foregoing description and in the following examples may, both separately or in any combination thereof, be material for realising the invention in diverse forms thereof.

EXAMPLES

Example 1

*Materials* - Reduced and oxidized glutathione (GSH and GSSG, respectively), cysteine (Cys), DL-homocysteine (hCy), cysteinylglycine (CG), and y-glutamylcysteine (y-GC) were purchased from Sigma. Cysteamine (Cya) and 7-fluorobenzofurazan-4-sulfonic acid ammonium salt (SBD-f) were obtained from Fluka. Tris(2-carboxyethyl)phosphine (TCEP) was purchased from Calbiochem (Merck KGaA, Darmstadt, Germany). Chromogenic S-2288 substrate was obtained from Chromogenix (Milano, Italy). PEG5k-maleimide (2E2M0H01), PEG20k-maleimide (2E2M0P01), PEG40k-maleimide (2D3Y0T01), and maleimide-PEG3.4k-maleimide (2E2E0F02) were purchased from Nektar Therapeutics (Huntsville, AL). d-Phe-Phe-Arg-chloromethyl ketone was purchased from Bachem. Triphenylphosphine-3,3', 3" trisulfonic acid was obtained from Aldrich. Human plasma-derived FX and FXa were obtained from Enzyme Research Laboratories Inc. (South Bend, IN). Biotin-polyethyleneoxide-iodoacetamide (Biotin-PEO-iodoacetamide) came from Sigma (Missouri, USA). Soluble tissue factor 1-219 (sTF) was prepared according to published procedures (Freskgard

et al., 1996). sTF (1-219) Glu219→Cys (E219C) was prepared essentially as described previously (Owenius et al., 1999). Expression and purification of recombinant FVIIa was performed as described previously (Thim et al., 1988; Persson and Nielsen, 1996). All other chemicals were of analytical grade or better.

*Concentration determination* - The concentration of GSSG in stock solutions was determined from its absorption at 248 nm using an extinction coefficient of 381 $M^{-1}cm^{-1}$ (Chau and Nelson, 1991). The concentration of GSH and other low-molecular weight thiols were determined using Ellman's reagent (5,5'-dithiobis(2-nitrobenzoic acid)) and 14150 $M^{-1}cm^{-1}$ as the molar extinction coefficient of 2-nitro-5-thiobenzoic acid at 412 nm (Riddles et al., 1979).

*Quantification of GSSG by HPLC* - Quantification of GSSG was performed essentially as described elsewhere (Takahashi and Creighton, 1996). Briefly, 50 μl acid quenched sample were loaded onto a $C_{18}$ reversed-phase column (Luna C18(2) 100 Å, 3 μm particle size, 4.6x50mm; Phenomenex Inc., Torrance, CA) maintained at 30°C. Following 5 min isocratic run at 100% eluent A (0.1% (v/v) trifluoroacetic acid (TFA) in water), GSSG was eluted by a linear gradient from 0-5% eluent B (0.085% (v/v) TFA in acetonitrile) in 5 min at a flow rate of 1 ml/min and detected by absorption at 214 nm. The concentration of GSSG was determined by relating the calculated peak area (Millenium32 v4.0 software, Waters) to a standard curve made with known amounts of GSSG. Linearity was observed in the range from 2-25 nmol GSSG.

*Analysis of thiol-modified FVIIa 407C by HPLC* - Free and thiol-modificed FVIIa 407C species were analyzed by HPLC using a $C_3$ reversed-phase column (Zorbax 300SB-C3, 2.1x150mm, 5-μm particle size; Agilent Technologies, Denmark) maintained at 45°C. The flow rate was 0.5 ml/min and mobile phases consisted of 0.1% (v/v) TFA in water (eluent A) and 0.085% (v/v) TFA in acetonitrile (eluent B). After injection of 25 μl acid quenched sample, the system was run isocratically at 30% eluent B for 5 min followed by linear gradients from 38-41.5% eluent B over 20 min and 41.5-55% eluent B over 20 min. The eluate was monitored by fluorescence (excitation and emission wavelengths of 280 and 348 nm, respectively).

*Construction of DNA encoding FVII 407C mutant* - A DNA construct encoding FVIIa 407C was constructed as described in WO 02/077218 A1

*Construction of DNA encoding FVII Q250C mutant* - A DNA construct encoding FVIIa Q250C was constructed as described in WO 02/077218 A1

*Construction of DNA encoding FVII R396C mutant* - A DNA construct encoding FVIIa R396C was constructed as described in WO 02/077218 A1

*Expression and purification of FVII 407C* - BHK cells were transfected essentially as previously described (Thim et al., 1988; Persson and Nielsen, 1996) to obtain expression of the FVIIa 407C variant. The Factor VII polypeptide was purified as follows:

Conditioned medium was loaded onto a 25-ml column of Q Sepharose Fast Flow (Amersham Biosciences, GE Healthcare) after addition of 5 mM EDTA, 0.1% Triton X-100 and 10 mM Tris, adjustment of pH to 8.0 and adjustment of the conductivity to 10-11 mS/cm by adding water. Elution of the protein was accomplished by a gradient from 10 mM Tris, 50 mM NaCl, 0.1% Triton X-100, pH 8.0 to 10 mM Tris, 50 mM NaCl, 25 mM $CaCl_2$, 0.1% Triton X-100, pH 7.5. The fractions containing FVIIa 407C were pooled, and applied to a 25-ml column containing the monoclonal antibody F1A2 (Novo Nordisk A/S, Bagsværd, Denmark) coupled to CNBr-activated Sepharose 4B (Amersham Biosciences, GE Healthcare). The column was equilibrated with 50 mM HEPES, pH 7.5, containing 10 mM $CaCl_2$, 100 mM NaCl and 0.02% Triton X-100. After washing with equilibration buffer and equilibration buffer containing 2 M NaCl, bound material was eluted with equilibration buffer containing 10 mM EDTA instead of $CaCl_2$. Before storage, FVIIa 407C was transferred to a 50 mM HEPES, 100 mM NaCl, 10 mM $CaCl_2$, pH 7.0 buffer by dialysis. The yield of each step was followed by factor VII ELISA measurements and the purified protein was analysed by SDS-PAGE.

*Expression and purification of FVII Q250C* - BHK cells were transfected essentially as previously described (Thim et al., 1988; Persson and Nielsen, 1996) to obtain expression of the FVIIa Q250C variant. The Factor VII polypeptide was purified as follows:

Conditioned medium was loaded onto a 25-ml column of Q Sepharose Fast Flow (Amersham Biosciences, GE Healthcare) after addition of 5 mM EDTA, 0.1% Triton X-100 and 10 mM Tris, adjustment of pH to 8.0 and adjustment of the conductivity to 10-11 mS/cm by adding water. Elution of the protein was accomplished by a gradient from 10 mM Tris, 50 mM NaCl, 0.1% Triton X-100, pH 8.0 to 10 mM Tris, 50 mM NaCl, 25 mM $CaCl_2$, 0.1% Triton X-100, pH 7.5. The fractions containing FVIIa 407C were pooled, and applied to a 25-ml column containing the monoclonal antibody F1A2 (Novo Nordisk A/S, Bagsværd, Denmark) coupled to CNBr-activated Sepharose 4B (Amersham Biosciences, GE Healthcare). The column was equilibrated with 50 mM HEPES, pH 7.5, containing 10 mM $CaCl_2$, 100 mM NaCl and 0.02% Triton X-100. After washing with equilibration buffer and equilibration buffer containing 2 M NaCl, bound material was eluted with equilibration buffer containing 10 mM EDTA instead of $CaCl_2$. Before

storage, FVIIa Q250C was transferred to a 50 mM HEPES, 100 mM NaCl, 10 mM $CaCl_2$, pH 7.0 buffer by dialysis. The yield of each step was followed by factor VII ELISA measurements and the purified protein was analysed by SDS-PAGE.

*Expression and purification of FVII R396C* - Expression and purification of FVIIa R396C was performed as described in WO 02/077218 A1

*Cloning and expression of glutaredoxins* - DNA coding sequences for *Escherichia coli* glutaredoxin 2 (Grx2) and *Saccharomyces cerevisiae* glutaredoxin 1 (yGrx1p) were amplified by PCR using Expand High Fidelity PCR system (Roche Diagnostics Corporation, Indianapolis, IN) according to manufacturer's recommendations and primer pairs oHOJ98-f/oHOJ98-r and oHOJ11-f/oHOJ11-r, respectively, introducing flanking *Nde*I and *Xho*I restriction sites (primer sequences are listed in Table 1).

**Table 1** - DNA oligos used for construction of plasmids pHOJ294, 210, and 286 expressing *E.coli* glutaredoxin 2 (Grx2), *S.cerevisiae* glutaredoxin 1 (yGrx1p), and yGrx1p C30S, respectively. *Nde*I and *Xho*I restriction sites are shown in bold face.

| Primer | Plasmid | Target | Sequence (5'→3') |
|---|---|---|---|
| oHOJ11-f | pHOJ210 | yGrx1p | GGGCCGCC**CATATG**GTATCTCAAGAAACTATC |
| oHOJ11-r | pHOJ210 | yGrx1p | GCCCGGG**CTCGAG**ATTTGCAAGAATAGGTTCTAAC |
| oHOJ98-f | pHOJ294 | Grx2 | GCCGCCGG**CATATG**AAGCTATACATTTACGATCACTGCCC |
| oHOJ98-r | pHOJ294 | Grx2 | CCGCCGCC**CTCGAG**AATCGCCATTGATGATAACAAATTGATTTGTG |
| oHOJ88-f | pHOJ286 | yGrx1p C30S | GTTTAGGGCTGCATGCGAGTATGGACAGTACG |
| oHOJ88-r | pHOJ286 | yGrx1p C30S | CGTACTGTCCATACTCGCATGCAGCCCTAAAC |

Genomic template DNA for PCR reactions was prepared from *E.coli* and *S.cerevisiae* according to published procedures (Grimberg et al., 1989; Hoffman and Winston, 1987). The purified PCR products were cut with NdeI and XhoI and then ligated into the corresponding sites of pET-24a(+) (Novagen) to give pHOJ294 and pHOJ210, respectively. Since stop codons were provided by the vector, the two genes were equipped with 3' vector-derived extensions encoding C-terminal LEHHHHHH affinity tags. Plasmid pHOJ286 encoding yGrx1p Cys30Ser (yGrx1p C30S) was constructed by QuickChange® Site-Directed Mutagenesis using primers oHOJ88-f/oHOJ88-r and pHOJ210 as template according to manufacturer's instructions (Stratagene, La Jolla, CA). The correct identity of all cloned sequences was verified by DNA sequencing.

For expression, pHOJ210, 286, and 294 plasmids were introduced into chemical competent BL21(DE3) cells (Stratagene, La Jolla, CA). Fresh overnight transformants were inoculated into 500 ml terrific broth ((Sambrook et al., 1989)) and 30 μg/ml kanamycine to an initial $OD_{600}$ of 0.02. Cultures were grown at 37°C in baffled flasks at 230 rpm to the mid-log phase ($OD_{600}$ 3-4) at which time the temperature was lowered to 25°C and protein expression induced by 0.1 mM isopropyl-β-D-thiogalactopyranoside (ITPG). After overnight expression, cells were harvested by centrifugation, resuspended in 50 ml lysis buffer (50 mM potassium phosphate, 300 mM NaCl, pH 8.0), and lysed by three freeze-thaw cycles. The cleared lysate was loaded onto a 20-ml Ni-NTA Superflow (Qiagen GmbH, Hilden, Germany) column equilibrated with lysis buffer at a flow rate of 5 ml/min. After washing with lysis buffer, bound protein was eluted with a linear gradient from 0-200 mM imidazole in lysis buffer. Peak fractions were pooled, treated with 20mM dithiothreitol for 20 min before extensive dialysis against 50 mM Tris-HCl, 2 mM EDTA, pH 8.0. Proteins were stored at -80°C and judged to be >90% pure by SDS-PAGE. Concentrations were estimated by absorbance at 280 nm using extinction coefficients of 5240 $M^{-1}cm^{-1}$ (yGrx1p and yGrx1p C30S) and 21740 $M^{-1}cm^{-1}$ (Grx2).

*Identification of low-molecular weight thiols engaging in mixed disulfides with FVIIa 407C* - HPLC detection of fluorescent SBD-derivatized low-molecular weight thiols was performed as described by Oe et al. (1998) with minor modifications. Briefly, disulfide reduction and subsequent derivatization of liberated thiols was performed by incubating 25 μl of 10 μM FVIIa 407C (or wild-type FVIIa) in 160 mM Tris-HCl, 8 mM EDTA, pH 9.6 buffer with 5 μl 14 mM TCEP (in water) and 10 μl 0.3% SBD-f (in water) at 60°C for 60 min. Subsequently, derivatization was terminated by addition of 2 μl 5 M HCl and samples were placed at 4°C until further analysis (within 24 hr). HPLC analysis was performed by injecting 25-μl aliquots of the samples onto a reversed-phase C18(2) column (Luna, 100 Å, 3.5 μm particle size, 150x4.6 mm; Phenomenex Inc., Torrance, CA) at a flow rate of 1 ml/min. The column temperature was maintained at 30°C. SBD-derivatized thiols were separated by isocratic elution using a mobile phase consisting of 75 mM Na-Citrate, pH 2.90 and

2% methanol and detected by the fluorescence emitted at 516 nm upon excitation at 386 nm. Peak identification was performed by comparison of retention times with those of a series of known low-molecular weight thiol compounds prepared according to the procedure described above for FVIIa 407C. Calibration curves for quantification of GSH, γ-GC, GC, Cys, Hcy, and Cya were obtained by varying the concentration of each thiol from 0.4 to 3.5 μM in the final reaction mixture.

From this analysis, it can be concluded that major low-molecular weight thiols conjugated to FVIIa 407C are glutathione, cysteine, and homocysteine. Results are presented in Figure 1.

*Redox titration of FVIIa* - To identify conditions appropriate for selective reduction of FVIIa Cys mutants, the structural stability of FVIIa was assessed in buffers with defined redox potentials obtained by varying concentrations of GSH and GSSG essentially as described elsewhere (Loferer et al., 1995). Since reduction of the two most labile disulfide bonds in FVIIa has been shown to be associated with a loss of amidolytic activity and sTF binding (Higashi et al., 1997), the structural integrity of FVIIa was monitored by its ability to hydrolyse the chromogenic substrate S-2288 in the presence of sTF.

Redox titration of FVIIa (1 μM) was performed in 50 mM HEPES, 100 mM NaCl, 5 mM $CaCl_2$, pH 7.0 buffer (thoroughly purged with nitrogen) containing 50 μM GSSG and varying concentrations of GSH (0 - 34 mM). In addition, one series of samples contained 25 mM *p*-aminobenzamidine, an active-site inhibitor of FVIIa occupying the $S_1$ pocket (Sichler et al., 2002; Persson et al., 2004). To reduce the time required to reach equilibrium, reactions were performed in the presence of 1 μM yGrx1p acting as a redox catalyst (Ostergaard et al., 2004). After equilibration of the samples for 3.5 hours at 30°C under nitrogen atmosphere, residual amidolytic activity was determined as described below. At the same time, an aliquot of the reaction mixture was quenched by an equal volume of 100 mM HCl, and the equilibrium concentration of GSSG determined by HPLC as described in *materials and methods.*

For measurement of residual amidolytic activity, 20 μl of the equilibrated samples were diluted 20-fold into assay buffer (50 mM HEPES, 100 mM NaCl, 5 mM $CaCl_2$, 0.01% Tween 80, pH 7.4) containing 10 mM iodoacetamide to rapidly alkylate free thiols and prevent subsequent thiol oxidation. The activity assay was carried out in polystyrene microtiter plates (Nunc, Denmark) in a final volume of 200 μl assay buffer containing 80 nM sTF and quenched sample to a final concentration of 10 nM FVIIa. After 15 min pre-incubation at room temperature, 1 mM chromogenic substrate S-2288 was added and the absorbance monitored continuously at 405 nm for 20 min in a Spectramax™ 340 microplate spectrophotometer equipped with SOFTmax PRO software (v2.2; Molecular Devices Corp., Sunnyvale, CA). Amidolytic activity was reported as the slope of the linear progress curves after blank subtraction.

Data were analyzed in terms of the following reaction (Eq. 1), where FVIIa is converted into inactivated FVIIa (denoted FVIIai) by reversible reduction of a single intramolecular disulfide bond:

Eq. 1 $$\text{FVIIa} + 2\text{GSH} \Leftrightarrow \text{FVIIai} + \text{GSSG}$$

The apparent equilibrium constant for the reverse reaction ($K_{ox}$) can be estimated from the following relationship (Eq. 2)

Eq. 2 $$f = a_{max}/(1+[\text{GSH}]^2/([\text{GSSG}]\ K_{ox}))$$

where f is the residual amidolytic activity at a given $[GSH]^2/[GSSG]$, and $a_{max}$ is the limiting amidolytic activity at low $[GSH]^2/[GSSG]$.

Fitting the redox titration data to Eq. 2 by non-linear least squares regression using Kaleidagraph software (v3.6, Synergy software) yielded apparent $K_{ox}$'s of 93±6 mM and 166±16 mM in the absence or presence of 25 mM p-aminobenzamidine, respectively (Figure 2).

*Redox titration of FVIIa 407C-glutathione mixed disulfide* - The stability of the mixed disulfide between glutathione and Cys407 was measured by incubating 13 μM FVIIa 407C in 50 mM HEPES, 100 mM NaCl, 10mM $CaCl_2$, pH 7.0 containing 0.5 mM GSH and varying concentrations of GSSG (5-500 μM). In addition, all samples contained 10 μM Grx2 to catalyze the reaction. After 5 hours equilibration at 30°C, a 50-μl aliquot was quenched with 100 mM HCl and the equilibrium concentration of GSSG determined by HPLC as described in *materials and methods.* To measure the relative amount of deglutathionylated FVIIa 407C, free thiols were labelled with PEG5k by combining 20 μl of each sample with 15 μl 1.6 mM PEG5k-maleimide. Following 18 min incubation at room temperature, N-ethylmaleimide was added to a final concentration of 25 mM to competitively suppress further (unspecific) PEGylation of the protein during subsequent processing. PEG5k-modified FVIIa 407C in each sample was detected and quantified by HPLC as described in *material and methods.*

Data were analyzed according to the following reaction (Eq. 3), where glutathionylated FVIIa 407C (FVIIa 407C-GSH) reacts with GSH to give free FVIIa 407C and GSSG

$$\text{Eq. 3} \qquad \text{FVIIa 407C-GSH + GSH} \Leftrightarrow \text{FVIIa 407C + GSSG}$$

The apparent equilibrium constant for the reverse reaction, denoted $K_{scox}$, can be estimated from the following relationship (Eq. 4)

$$\text{Eq. 4} \qquad A_{407C\text{-}PEG5k} = A_{max}([GSH]/[GSSG])/([GSH]/[GSSG] + K_{scox})$$

where $A_{407C\text{-}PEG5k}$ is the peak area of 5k-PEGylated FVIIa 407C at a given [GSH]/[GSSG] ratio, and $A_{max}$ is the limiting peak area at high [GSH]/[GSSG].

A plot of the measured peak areas versus the [GSH]/[GSSG] ratio at equilibrium is shown in Figure 3. Fitting of Eq. 4 to the data by non-linear least squares regression using Kaleidagraph software (v3.6, Synergy software) gave an apparent $K_{scox}$ of 1.0, very similar to that measured for a range of other glutathionylated proteins (Gilbert, 1995).

*Identification of optimal reduction conditions* - Optimal glutathione redox conditions supporting selective reduction of the FVIIa 407C- mixed disulfides were identified from plots of the following parameters as a function of [GSSG]: (1) the residual amidolytic activity in the presence or absence ofp-aminobenzamidine using Eq. 2 and estimated $K_{ox}$ values, and (2) the fraction of selectively reduced protein from Eq. 4 and $K_{scox}$. For practical reasons, the concentration of GSH was set to 0.5 mM. As shown in Figure 4, a concentration of GSSG between roughly 15 and 60 μM in the presence of 0.5 mM GSH results in >90% residual activity and >90% free Cys407. The optimal [GSSG] working range depends on several parameters, including the concentration of GSH (as exemplified in Figures 3-5), the values of $K_{ox}$ and $K_{scox}$ (not shown), and the allowed loss of amidolytic activity during the reduction reaction.

*Selective reduction and PEG5k, PEG20k, and PEG40k modification of FVIIa 407C* - Thiol modification of FVIIa 407C can be divided into three consecutive steps: (A) a glutaredoxin-catalyzed reduction reaction, (B) thiol-specific alkylation, and (C) purification. At the end of each step, a small aliquot of the reaction mixture was quenched with 10% (v/v) formic acid and analyzed by HPLC as described in *material and methods* and exemplified in Figure 7.

(A) FVIIa 407C (4.8 mg) was incubated 4.5 hours at 30°C in a total volume of 4.4 ml 50 mM HEPES, 100 mM NaCl, 10 mM $CaCl_2$, pH 7.0 buffer containing 0.5 mM GSH, 15 μM GSSG, 25 mM p-aminobenzamidine, and 10 μM Grx2. The initial concentration of GSSG was in the lower end of the optimal working range (shaded area in Figure 4) to compensate for the formation of GSSG during the reaction. (B) Subsequently, free thiols were modified by addition of PEG5k-maleimide, PEG20k-maleimide, or PEG40k-maleimide (dissolved in water) to a final concentration of 0.8 mM. Thiol alkylation was allowed to proceed for 15 min at room temperature upon quenching with 0.5 mM cysteine. (C) EDTA was added in excess of calcium (20 mM final concentration) and the entire content loaded onto a 1 ml HiTrap Q FF column (Amersham Biosciences, GE Healthcare) equilibrated with buffer A (50 mM HEPES, 100 mM NaCl, 1mM EDTA, pH 7.0) to capture FVIIa 407C. After wash with buffer A, one-step elution of bound protein was performed with buffer B (10 mM GlyGly, 150 mM NaCl, 10 mM $CaCl_2$, 0.01% Tween 80, pH 7.0) directly onto a HiLoad Superdex 200 16/60 pg column (Amersham Biosciences) mounted in front of the HiTrap column. PEGylated and non-PEGylated species were separated at a flow rate of 1 ml/min and detected by absorption at 280 nm.

*Selective reduction and PEG3.4 or PEG20k-crosslinking of FVIIa 407C* - Selective reduction was carried out by incubating 4.8 mg FVIIa 407C at 30°C for 4.5 hours in a total volume of 4.4 ml 50 mM HEPES, 100 mM NaCl, 10 mM $CaCl_2$, pH 7.0 buffer containing 0.5 mM GSH, 10 μM GSSG, 25 mM p-aminobenzamidine, and 10 μM Grx2. Then, EDTA was added in excess of calcium (20 mM final concentration) and the entire content loaded onto a 1 ml HiTrap Q FF column (Amersham Biosciences, GE Healthcare) equilibrated in buffer A (50 mM HEPES, 100 mM NaCl, 1mM EDTA, pH 7.0) to capture FVIIa 407C. After wash with buffer A to remove unbound glutathione buffer and Grx2p, FVIIa 407C was eluted in one step with buffer B (50 mM HEPES, 100 mM NaCl, 10 mM $CaCl_2$, pH 7.0). The concentration of FVIIa 407C in the eluate was measured by absorbance at 280 nm using an extinction coefficient of $62 \cdot 10^3$ $M^1cm^{-1}$. Cross-linking was performed in the presence of approximately 0.6 equivalent of maleimide-PEG3.4k-maleimide or maleimide-PEG20k-maleimide for 1.5 hours at room temperature. (C) EDTA was added in excess of calcium (20 mM final concentration) and the entire content loaded onto a 1 ml HiTrap Q FF column (Amersham Biosciences, GE Healthcare) equilibrated in buffer A (50 mM HEPES, 100 mM NaCl, 1mM EDTA, pH 7.0) to capture FVIIa 407C. After wash with buffer A, one-step elution of bound protein was performed with buffer B (10 mM GlyGly, 150 mM NaCl, 10 mM $CaCl_2$, 0.01% Tween 80, pH 7.0) directly onto a HiLoad Superdex 200 16/60 pg column (Amersham Biosciences) to separate PEGylated and non-PEGylated species. The flow rate was 1 ml/min and protein was detected by absorption at 280 nm.

*SDS-PAGE analysis of FVIIa 407C, FVIIa 407C-PEG5k, FVIIA 407C-PEG20k, FVIIa 407C-PEG40k, and FVIIA 407C-PEG3.4k-FVIIa 407C* - FVIIa 407C and 5k, 20k, 40k, and 3.4k-PEGylated compounds (approx. 1.5 µg of each) were analyzed by reducing and non-reducing SDS-PAGE on a 4-12% Bis-Tris NuPAGE® gel (Invitrogen Life Technologies, Carlsbad, CA) run at 200 V for 35 min in MES buffer (Invitrogen Life Technologies, Carlsbad, CA) according to manufacturer's recommendations. Gels were washed with water and stained with Simply Blue™ SafeStain (Invitrogen Life Technologies, Carlsbad, CA) according to manufacturer's recommendations. Gels are shown in Figure 8.

*SDS-PAGE analysis of FVIIa 407C, FVIIA 407C-PEG3.4k-FVIIa 407C, and FVIIa 407C-PEG20k-FVIIa 407C* - Proteins (approx. 1 µg of each) were analyzed by reducing and non-reducing SDS-PAGE on a 4-12% Bis-Tris NuPAGE® gel (Invitrogen Life Technologies, Carlsbad, CA) run at 200 V for 35 min in MES buffer (Invitrogen Life Technologies, Carlsbad, CA) according to manufacturer's recommendations. Gels were washed with water and stained with Simply Blue™ SafeStain (Invitrogen Life Technologies, Carlsbad, CA) according to manufacturer's recommendations. Gels are shown in Figure 9.

*Active-site titration of FVIIa 407C, FVIIA 407C-PEG5k, FVIIa 407C-PEG20k, FVIIa 407C-PEG40k, FVIIA 407C-PEG3.4k-FVIIa 407C, and FVIIa 407C-PEG20k-FVIIa 407C* - Active site concentrations of FVIIa 407C and PEGylated compounds were determined from the irreversible loss of amidolytic activity upon titration with sub-stoichiometric levels of d-Phe-Phe-Arg-chloromethyl ketone (FFR-cmk) essentially as described elsewhere (Bock, 1992). Briefly, each protein was diluted into 50 mM HEPES, 100 mM NaCl, 10 mM $CaCl_2$, 0.01% Tween 80, pH 7.0 buffer to an approximate concentration of 300 nM using an extinction coefficient of $62 \cdot 10^3$ $M^{-1}cm^{-1}$ at 280 nm. Diluted protein (20 µl) was then combined with 20 µl 1.5 µM sTF and 20 µl 0-1.2 µM FFR-cmk (freshly prepared in buffer from a FFR-cmk stock dissolved in DMSO and stored at -80°C). After overnight incubation at room temperature, residual amidolytic activity was measured.

The activity assay was carried out in polystyrene microtiter plates (Nunc, Denmark) in a final volume of 200 µl assay buffer (50 mM HEPES, 100 mM NaCl, 5 mM $CaCl_2$, 0.01% Tween 80, pH 7.4) containing 50 nM sTF and approx. 10 nM FVIIa, corresponding to 10-fold dilutions of the samples. After 15 min pre-incubation at room temperature, 1 mM chromogenic substrate S-2288 was added and the absorbance monitored continuously at 405 nm for 20 min in a SpectraMax™ 340 microplate spectrophotometer equipped with SOFTmax PRO software (v2.2; Molecular Devices Corp., Sunnyvale, CA). Amidolytic activity was reported as the slope of the linear progress curves after blank subtraction. Active site concentrations were determined by extrapolation, as the minimal concentration of FFR-cmk completely abolishing amidolytic activity.

In Table 2 are given the measured active site concentrations relative to the absorbances of the proteins at 280 nm. Values are normalized to 100% for FVIIa 407C.

**Table 2 -** Specific active-site concentrations of FVIIa 407C and PEGylated variants. Specific active-site concentrations were measured as the active-site concentration by FFR-cmk titration relative to the absorbance of the protein at 280 nm. Values are normalized to 100% for FVIIa 407C.

| **Protein** | **[Active-site]/$A_{280}$** |
|---|---|
| FVIIa 407C | 100% |
| FVIIa 407C-PEG5k | 90% |
| FVIIa 407C-PEG20k | 86% |
| FVIIa 407C-PEG40k | 91% |
| FVIIa 407C-PEG3.4k-FVIIa 407C | 95% |
| FVIIa 407C-PEG20k-FVIIa 407C | 90% |

*Surface plasmon resonance (SPR) analysis of the binding of FVIIa 407C, FVIIa 407C-PEG3.4k-FVIIa 407C, and FVIIa 407C-PEG20k-FVIIa 407C to immobilized soluble tissue factor* - Binding of FVIIa 407C and PEGylated variants to immobilized soluble tissue factor was analyzed by surface plasmon resonance analysis on a Biacore 3000 instrument (Biacore AB, Uppsala, Sweden). Prior to immobilization, sTF E219C was biotinylated at its free cysteine by reacting the protein with 2mM biotin-PEO-iodoacetamide at pH 7.0 for 20 min. Excess reagent was subsequently removed by gelfiltration on a NAP™5 column (Amersham Biosciences AB, Uppsala, Sweden) equilibrated in HBS-P buffer (10mM HEPES, 150mM NaCl, 0,005% P20; Biacore AB, Uppsala, Sweden) containing 5mM $CaCl_2$. Biotinylated sTF E19C was immobilized at a density of 2.2 fmol/$mm^2$ (~50 RU) in flow cell 2 of a SA sensor chip (Biacore AB, Uppsala, Sweden). Kinetic analysis was performed at a flow rate of 30 µl/min in running buffer (HBS-P buffer containing 5mM $CaCl_2$) using the untreated flow cell 1 for automatic in-line reference subtraction. Serial dilutions of protein from 100nM to approx. 5nM were analyzed. Following 3 min equilibration of the flow cells in running buffer, 90 µl protein sample was injected using the KINJECT command. The dissociation phase lasted 9 min and regeneration was performed with a 3-min pulse

of 20 mM EDTA in HBS-P buffer. SPR data were fitted to a 1:1 Langmuir binding model using BIAevaluation 4.1 software (Biacore AB, Uppsala, Sweden). Results are given in Table 3.

**Table 3** - Surface plasmon resonance analysis of the binding of FVIIa 407C and dimeric variants to immobilized sTF. Dissociation constants ($K_D$) were calculated from the measured association ($k_{on}$) and dissociation ($k_{off}$) rate constants.

| **Protein** | $k_{on}$ ($M^{-1}s^{-1}$) | $k_{off}$ ($s^{-1}$) | $K_D$ |
|---|---|---|---|
| FVIIa 407C | $2.8 \times 10^5$ | $1.5 \times 10^{-3}$ | 5.5 nM |
| FVIIa 407C-PEG3.4k-FVIIa 407C | $3.5 \times 10^5$ | $1.5 \times 10^{-4}$ | 0.4 nM |
| FVIIa 407C-PEG20k-FVIIa 407C | $3.1 \times 10^5$ | $1.6 \times 10^{-4}$ | 0.5 nM |

*Reduction of FVIIa R396C-mixed disulfides using triphenylphosphine-3,3',3" trisulfonic acid* - Small-scale reduction of FVIIa R396C-mixed disulfides using triphenylphosphine-3,3',3" trisulfonic acid ($PPh_3S_3$) was performed as follows: FVIIa R396C (4.4 μM) was treated with either 2.5 or 5.0 mM $PPh_3S_3$ in a total volume of 50 μl reaction buffer (50 mM HEPES, 100 mM NaCl, 10 mM $CaCl_2$, 0.05% Tween 20, pH 7.0) containing 50 mM p-aminobenzamidine. After 16.3 hours incubation at room temperature, reaction mixtures (30 μl) were desalted on Pro●Spin™ Spin columns (Princeton Separations, Adelphia, New Jersey) rehydrated in reaction buffer according to manufacturer's instructions to remove excess reductant. Subsequently, free thiols were alkylated with 0.2 mM PEG5k-maleimide for 10 min at room temperature. PEGylated and non-PEGylated FVIIa R396C were separated by reducing SDS-PAGE on a 4-12% Bis-Tris gel (Invitrogen Life Technologies, Carlsbad, CA) run at 200 V for 35 min in MES buffer according to manufacturer's recommendations. Gel staining with Simply Blue™ SafeStain (Invitrogen Life Technologies, Carlsbad, CA) was performed according to manufacturer's instructions. The gel is shown in Figure 10A.

The amidolytic activity of FVIIa R396C before and after incubation with $PPh_3S_3$ was measured by 320-fold dilution of the reaction mixture into 200 μl (total volume) 50 mM HEPES, 100 mM NaCl, 5 mM $CaCl_2$, 1 mg/ml BSA, pH 7.4 buffer containing 50 mM sTF. After 15 min pre-incubation at room temperature, 1 mM chromogenic substrate S-2288 was added and the absorbance monitored continuously at 405 nm for 20 min in polystyrene microtiter plates (Nunc, Denmark) using a SpectraMax™ 340 microplate spectrophotometer equipped with SOFTmax PRO software (v2.2; Molecular Devices Corp., Sunnyvale, CA). Amidolytic activity was reported as the slope of the linear progress curves after blank subtraction. Results are shown in Figure 10B.

*Selective Reduction* of *Exposed Disulfides in a Factor VII polypeptide* - The commercially available triarylphosphine **1** (trisodium salt of triphenylphosphine-3,3',3"-trisulfonic acid from Aldrich) contains approximately 5% of the corresponding 3,3'-bis-sulfonic acid **2**. Thus, **1** was purified by standard reverse-phase HPLC, eluting with a gradient of acetonitrile in water in the presence of 0.1% trifluoroacetic acid.

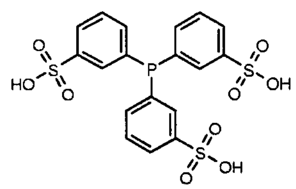
1

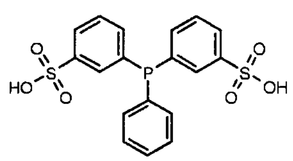
2

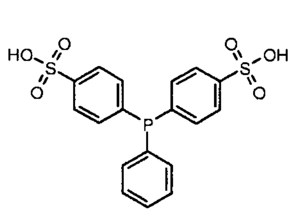
3

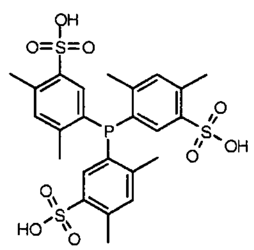
4

It has been shown that triphenylphosphine-3,3',3"-trisulfonic acid (2.5 mM) can be used in conjunction with the active site inhibitor 4-aminobenzamidine to reduce the exposed disulfide bond between glutathione and FVIIa R396C essentially without loss of amidolytic activity. Reductive cleavage of the mixed disulfide bond was demonstrated by subsequent modification of the liberated cysteine with PEG5k-maleimide.

Triarylphosphines **1**-**3** (10 mM) were individually incubated with rFVIIa for 1 h at room temperature. In the presence of **1**, rFVIIa retained most of its activity. In contrast, the 3,3'-bis-sulfonic acid **2** caused a rapid decrease in the enzyme's amidolytic activity, much like the analogous 4,4'-bis-sulfonic acid **3** (dipotassium salt from Aldrich), and was therefore not considered optimal for reduction of rFVIIa.

Furthermore, **1** was tested for its ability to reduce cystine dimethyl ester **5**. The reaction was conducted at room

temperature at 15 mM concentration of **1** in water. The substrate was present at 5 mM concentration. It was demonstrated by LC-MS analyses that the disulfide bond in **5** was reduced under the given conditions.

**Scheme 1**

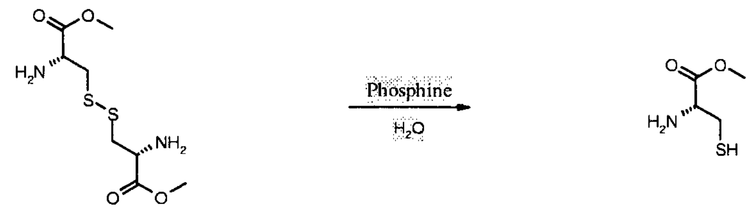


5 6

The more sterically hindered triarylphosphine **4** (trisodium salt from Strem) was found to be almost unreactive towards rfVIIa, suggesting the feasibility of developing a more selective reducing agent. Compounds **9**-**11** represent non-limiting examples of triarylphosphines which are expected to be selective disulfide reducing agents.

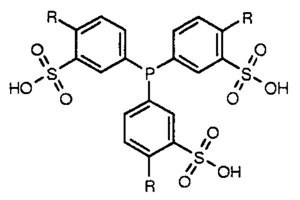

R = H, Me, Et, Pr, 2-Pr, Bu, MeO, EtO, PrO, 2-PrO, BuO.

9

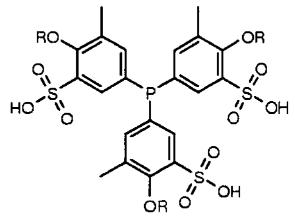

R = Me, Et, Pr, 2-Pr, Bu.

10

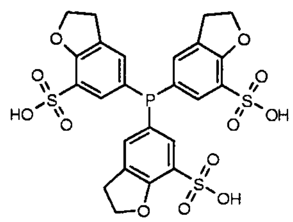

11

Example 2

*Materials* - FVIII deficient plasma was obtained from George King Bio-Medical Inc. (Kansas, USA; Prod. no. 0800). Lyophilised human thrombocytes were purchased from Helena Biosciences (UK, Prod. no. 5371). Thrombocyte reconstitution buffer: Tris Buffered Saline was obtained from Helena Biosciences (UK; Prod. no. 5365). Innovin was obtained from Dade Behring (Liederbach, Germany; Prod. no. B4212-50). Thrombin specific substrate: Z-Gly-Gly-Arg-AMC HCl Fluorophor was obtained from Bachem (Weil am Rhein, Germany; Prod. no. I-1140). Expression and purification of recombinant FVIIa (rFVIIa) was performed as described previously (Thim et al., 1988; Persson and Nielsen, 1996). All other chemicals were of analytical grade or better.

*Determination of rFVIIa-like activity of FVII-analogues using an Endogenous Thrombin Potential* (*ETP*) *bioassay* - The endogenous thrombin potential (ETP) assay is based on a real time determination of thrombin generation in a selected plasma sample. The plasma sample contains lyophilized thrombocytes as a physiological lipid surface source for the assembly of the Xase and prothrombinase complexes in the coagulation cascade. The real time thrombin activity is determined by continuous detection of an appearing fluorescent product from a thrombin specific substrate (Hemker et al., 2000; Hemker & Béguin, 2000).

The concentration of FVIIa and PEGylated variants were determined by absorbance measurements at 280 nm. A value of 1.36 was taken as the absorbance of a 1 mg/ml protein solution (E0.1%).

One vial of lyophilized thrombocytes was dissolved in 0.73 ml reconstitution buffer (Tris Buffered Saline) and further diluted to 75.000 thrombocytes/μl in FVIII deficient plasma. Innovin, recombinant FVIIa (rFVIIa) as well as test samples were diluted in assay buffer (20 mM Hepes, 150 mM NaCl, pH 7.35, 1.5% bovine serum albumin (BSA)). Thrombin specific substrate, Z-Gly-Gly-Arg-AMC, was dissolved in 60% dimethylsulfoxide (DMSO) and further diluted in calcium-containing assay buffer (100mM $CaCl_2$, 20 mM Hepes, 150 mM NaCl, pH 7.35). In a Costar microtiter plate (96 wells, Prod. No. 3631) 10 μl rFVIIa (final 0 - 10000 ng/ml), or test sample, or blank (assay buffer) (10 μl) were added to respective wells. Innovin (10 μl, final 0.12 pM), and thrombocyte containing plasma (80 μl, final 50.000 thrombocytes/μl) were added to respective wells of a Costar plate (96 wells, Prod. No. 3631). The plate was incubated for 10 min at 37°C in Thermo Fluoroscan Ascent (Thermo Electron Corporation, Cambridge, UK). Substrate (20 μl, final concentrations: 0.4 mM Thrombin specific substrate, 0.5% DMSO and 16.7 mM $CaCl_2$) was immediately added and fluorescence was continuously recorded for 60 minutes (excitation 390 nm, emission 460 nm).

Concentration-response curves were generated for rFVIIa and FVII-analogues based on the signal (Area under the fluorescent unit-time curve for 60 min). The ETP activity of the analogue relative to rFVIIa was calculated from the curves and given in Table 4.

**Table 4 -** ETP activities of dimeric FVIIa 407C molecules relative to rFVIIa. The relative activities are given per FVIIa 407C protomer and, thus, the total activities of the dimeric molecules are twice the reported values.

| Protein | ETP relative activity |
|---|---|
| FVIIa 407C-PEG3.4k-FVIIa 407C | 620% |
| FVIIa 407C-PEG20k-FVIIa 407C | 270% |

*References*

Bock,P.E. (1992). Active-site-selective labeling of blood coagulation proteinases with fluorescence probes by the use of thioester peptide chloromethyl ketones. I. Specificity of thrombin labeling. J Biol Chem 267, 14963-14973.

Chau,M.H. and Nelson,J.W. (1991). Direct measurement of the equilibrium between glutathione and dithiothreitol by high performance liquid chromatography. FEBS Lett. 291, 296-298.

Fernandes, A.P. and Holmgren, A. (2004) Glutaredoxins: glutathione-dependent redox enzymes with functions far beyond a simple thioredoxin backup system. Antioxid.Redox.Signal., 6, 63-74

Freskgard,P.O., Olsen,O.H., and Persson,E. (1996). Structural changes in factor VIIa induced by Ca2+ and tissue factor studied using circular dichroism spectroscopy. Protein Sci 5, 1531-1540.

Gilbert,H.F. (1995). Thiol/disulfide exchange equilibria and disulfide bond stability. Methods Enzymol. 251, 8-28.

Grant,C. (2001). MicroReview: Role of the glutathione/glutaredoxin and thioredoxin systems in yeast growth and response to stress conditions. Mol. Microbiol. 39, 533-541

Grimberg,J., Maguire,S., and Belluscio,L. (1989). A simple method for the preparation of plasmid and chromosomal E. coli DNA. Nucleic Acids Res 17, 8893.

Harvey et al. (2003), J. Biol. Chem., 278, 8363-8369.

Hemker, H. C. Giesen, P. L. Ramjee, M. Wagenvoord, R. and Beguin, S.. The thrombogram: monitoring thrombin generation in platelet-rich plasma. Thromb.Haem. 83 (4):589-591, 2000.

Hemker, H. C. and Beguin, S.. Phenotyping the clotting system. Thromb.Haem. 84 (5):747-751, 2000.

Higashi,S., Matsumoto,N., and Iwanaga,S. (1997). Conformation of factor VIIa stabilized by a labile disulfide bond (Cys-310-Cys-329) in the protease domain is essential for interaction with tissue factor. J. Biol. Chem. 272, 25724-25730.

Holmgren,A., Åslund,F. (1995) Glutaredoxin, Method Enzymol. 252, 283-292

Hoffman,C.S. and Winston,F. (1987). A ten-minute DNA preparation from yeast efficiently releases autonomous plasmids for transformation of Escherichia coli. Gene 57, 267-272.

Loferer,H., Wunderlich,M., Hennecke,H., and Glockshuber,R. (1995). A bacterial thioredoxin-like protein that is exposed to the periplasm has redox properties comparable with those of cytoplasmic thioredoxins. J. Biol. Chem. 270, 26178-26183.

Luikenhuis,S., Perrone,G., Dawes,I.W., and Grant,C.M. (1998). The yeast Saccharomyces cerevisiae contains two glutaredoxin genes that are required for protection against reactive oxygen species. Mol. Biol. Cell 9, 1081-1091

Lundberg,M., Johansson,C., Chandra,J., Enoksson,M., Jacobsson,G., Ljung,J., Johansson,M., and Holmgren,A. (2001). Cloning and expression of a novel human glutaredoxin (Grx2) with mitochondrial and nuclear isoforms. J Biol Chem 276, 26269-26275

Nelsestuen et al. (2001). J. Biol. Chem. 276, 39825-39831.

Rodriguez-Manzaneque,M.T., Ros,J., Cabiscol,E., Sorribas,A., and Herrero,E. (1999). Grx5 glutaredoxin plays a central role in protection against protein oxidative damage in Saccharomyces cerevisiae. Mol Cell Biol 19, 8180-8190

Oe,T., Ohyagi,T., Naganuma,A. (1998) Determination of γ-glutamylglutathione and other low-molecular-mass thiol compounds by isocratic high-performance liquid chromatography with fluorimetric detection. J. Chrom. B, 708, 285-289

Ostergaard,H., Tachibana,C., and Winther,J.R. (2004). Monitoring disulfide bond formation in the eukaryotic cytosol. J Cell Biol 166, 337-345.

Owenius,R.I, Osterlund,M., Lindgren,M., Svensson,M., Olsen,O.H., Persson,E., Freskgård,P.-O., Carlsson,U. (1999) Properties of spin and fluorescent labels at a receptor-ligand interface. Biophys. J. 77, 2237-2250

Padilla,C.A., Martinez-Galisteo,E., Barcena,J.A., Spyrou,G., and Holmgren,A. (1995). Purification from placenta, amino acid sequence, structure comparisons and cDNA cloning of human glutaredoxin. Eur J Biochem 227, 27-34

Persson et al. (2001). J. Biol. Chem. 276, 29195-29199.

Persson et al. (2001). Proc. Natl. Acad. Sci., 98, 13583-13588.

Persson et al. (2002), Eur. J. Biochem., 379, 497-503.

Persson,E., Bak,H., Ostergaard,A., and Olsen,O.H. (2004). Augmented intrinsic activity of Factor VIIa by replacement of residues 305, 314, 337 and 374: evidence of two unique mutational mechanisms of activity enhancement. Biochem J 379, 497-503.

Persson,E. and Nielsen,L.S. (1996). Site-directed mutagenesis but not gamma-carboxylation of Glu-35 in factor VIIa affects the association with tissue factor. FEBS Lett 385, 241-243.

Riddles,P.W., Blakeley,R.L., and Zerner,B. (1979). Ellman’s reagent: 5,5’-dithiobis(2-nitrobenzoic acid)--a reexamination. Anal. Biochem. 94, 75-81.

Sambrook,J., Fritsch,E.F., and Maniatis,T. (1989). Molecular Cloning: A Laboratory Manual. (Cold Spring Harbor, NY: Cold Spring Harbor Laboratory).

Shah et al. (1998). Proc. Natl. Acad. Sci., 95, 4229-4234.

Sichler,K., Banner,D.W., D’Arcy,A., Hopfner,K.P., Huber,R., Bode,W., Kresse,G.B., Kopetzki,E., and Brandstetter, H. (2002). Crystal structures of uninhibited factor VIIa link its cofactor and substrate-assisted activation to specific interactions. J. Mol. Biol. 322, 591-603.

Soejima et al. (2002). J. Biol. Chem. 277, 49027.

Takahashi,N. and Creighton,T.E. (1996). On the reactivity and ionization of the active site cysteine residues of Escherichia coli thioredoxin. Biochemistry 35, 8342-8353.

Thim,L., Bjoern,S., Christensen,M., Nicolaisen,E.M., Lund-Hansen,T., Pedersen,A.H., and Hedner,U. (1988). Amino acid sequence and posttranslational modifications of human factor VIIa from plasma and transfected baby hamster kidney cells. Biochemistry 27, 7785-7793.

Wang, E.C.W., Hung, S.-H., Cahoon,M., Hedstrom,L. (1997) The role of Cys191-Cys220 disulfide bond in trypsin: new targets for engineering substrate specificity. Protein Engineering, 10, 405-411

Yang,Y., Jao,S., Nanduri,S., Starke,D.W., Mieyal,J.J., and Qin,J. (1998). Reactivity of the human thioltransferase (glutaredoxin) C7S, C25S, C78S, C82S mutant and NMR solution structure of its glutathionyl mixed disulfide intermediate reflect catalytic specificity. Biochemistry 37, 17145-17156

## Claims

1. Dimeric or multimeric FVIIa compound comprising at least two FVIIa polypeptides covalently connected such as to retain the intrinsic catalytic activity of the FVIIa polypeptides, wherein said compound has an increased biological activity as compared to the biological activity of the constituent FVIIa polypeptides, and wherein said at least two FVIIa polypeptides are covalently connected via engineered cysteines in said FVIIa polypeptides.

2. The compound according to any one of the preceding claims, wherein two FVIIa polypeptides are covalently connected to form a dimeric FVIIa compound.

3. The compound according to any one of the preceding claims, wherein said FVIIa polypeptides covalently connected are identical FVIIa polypeptides.

4. The compound according to any one of the preceding claims, wherein at least two different FVIIa polypeptides are covalently connected.

5. The compound according to any one of the preceding claims, wherein said dimeric or multimeric FVIIa compound comprises a linker between the constituent FVIIa polypeptides.

6. The compound according to claim 5, wherein said linker has the structure that results from a reaction involving a bivalent cysteine reactive PEG.

7. The compound according to claim 5, wherein said linker has the structure that results from a reaction involving maleimide-PEG-maleimide.

8. The compound according to claim 7, wherein said maleimide-PEG-maleimide is selected from the group consisting of maleimide-PEG2kD-maleimide, maleimide-PEG3.4kD-maleimide, maleimide-PEG5kD-maleimide, maleimide-PEG10kD-maleimide, and maleimide-PEG20kD-maleimide.

9. The compound according to claim 5, wherein said linker comprises an amino acid sequence.

10. The compound according to any one of the preceding claims, wherein said FVIIa polypeptide is FVIIa 407C

11. The compound according to any one of the preceding claims, wherein said FVIIa polypeptide is a FVIIa 407C variant.

12. The compound according to any one of the preceding claims, wherein said FVIIa polypeptide is FVIIa P406C or a FVIIa P406C variant.

13. The compound according to any one of the preceding claims, wherein said FVIIa polypeptide is FVIIa R396C or a FVIIa R396C variant.

14. The compound according to any one of the preceding claims, wherein said FVIIa polypeptide is FVIIa Q250C or a FVIIa Q250C variant.

15. The compound according to any one of the preceding claims, wherein said FVIIa polypeptides are FVIIa variants having optimized GLA domains, e.g. combinations of Y4 (insertion), P10Q, K32E, D33F, D33E, and A34E.

16. The compound according to any one of the preceding claims, wherein said FVIIa polypeptides are FVIIa variants having increased intrinsic proteolytic activity, e.g. M298Q FVIIa, V158D/E296V/M298Q FVIIa, V158D/E296V/M298Q/K337A FVIIa, F374Y/L305V-FVlla, F374Y/L305V/S314E/K337A-FVIIa, F374Y/L305V/S314E-FVIIa, F374Y/L305V/K337A-FVIIa, L305V/K337A-FVIIa, V158D/E296V/M298Q/L305V-FVIIa, V158D/E296V/M298Q/L305V/K337A-FVIIa, V158T/M298Q-FVIIa, E296V/M298Q-FVIIa, V158D/E296V-FVIIa, V158D/M298Q-FVIIa and the 407C variants thereof.

**17.** The compound according to any one of claims 1-15, wherein said FVIIa variant is selected from the group consisting of L305V-FVIIa, L305V/M306D/D309S-FVIIa, L305I-FVIIa, L305T-FVIIa, F374P-FVIIa, V158T/M298Q-FVIIa, V158D/E296V/M298Q-FVIIa, K337A-FVIIa, M298Q-FVIIa, V158D/M298Q-FVIIa, L305V/K337A-FVIIa, V158D/E296V/M298Q/L305V-FVIIa, V158D/E296V/M298Q/K337A-FVIIa, V158D/E296V/M298Q/L305V/K337A-FVIIa, K157A-FVIIa, E296V-FVIIa, E296V/M298Q-FVIIa, V158D/E296V-FVIIa, V158D/M298K-FVIIa, S336G-FVIIa, L305V/K337A-FVIIa, L305V/V158D-FVIIa, L305V/E296V-FVIIa, L305V/M298Q-FVIIa, L305V/V158T-FVIIa, L305V/K337A/V158T-FVIIa, L305V/K337A/M298Q-FVIIa, L305V/K337A/E296V-FVIIa, L305V/K337A/V158D-FVIIa, L305V/V158D/M298Q-FVIIa, L305V/V158D/E296V-FVIIa, L305V/V158T/M298Q-FVIIa, L305V/V158T/E296V-FVIIa, L305V/E296V/M298Q-FVIIa, L305V/V158D/E296V/M298Q-FVIIa, L305V/V158T/E296V/M298Q-FVIIa, L305V/V158T/K337A/M298Q-FVIIa, L305V/V158T/E296V/K337A-FVIIa, L305V/V158D/K337A/M298Q-FVIIa, L305V/V158D/E296V/K337A-FVIIa, L305V/V158D/E296V/M298Q/K337A-FVIIa, L305V/V158T/E296V/M298Q/K337A-FVIIa, S314E/K316H-FVIIa, S314E/K316Q-FVIIa, S314E/L305V-FVIIa, S314E/K337A-FVIIa, S314E/V158D-FVIIa, S314E/E296V-FVIIa, S314E/M298Q-FVIIa, S314E/V158T-FVIIa, K316H/L305V-FVIIa, K316H/K337A-FVIIa, K316H/V158D-FVIIa, K316H/E296V-FVIIa, K316H/M298Q-FVIIa, K316H/V158T-FVIIa, K316Q/L305V-FVIIa, K316Q/K337A-FVIIa, K316Q/V158D-FVIIa, K316Q/E296V-FVIIa, K316Q/M298Q-FVIIa, K316Q/V158T-FVIIa, S314E/L305V/K337A-FVIIa, S314E/L305V/V158D-FVIIa, S314E/L305V/E296V-FVIIa, S314E/L305V/M298Q-FVIIa, S314E/L305V/V158T-FVIIa, S314E/L305V/K337A/V158T-FVIIa, S314E/L305V/K337A/M298Q-FVIIa, S314E/L305V/K337A/E296V-FVIIa, S314E/L305V/K337AN158D-FVIIa, S314E/L305VN158D/M298Q-FVIIa, S314E/L305V/V158D/E296V-FVIIa, S314E/L305VN158T/M298Q-FVIIa, S314E/L305V/V158T/E296V-FVIIa, S314E/L305V/E296V/M298Q-FVIIa, S314E/L305V/V158D/E296V/M298Q-FVIIa, S314E/L305V/V158T/E296V/M298Q-FVIIa, S314E/L305V/V158T/K337A/M298Q-FVIIa, S314E/L305V/V158T/E296V/K337A-FVIIa, S314E/L305V/V158D/K337A/M298Q-FVIIa, S314E/L305V/V158D/E296V/K337A-FVIIa, S314E/L305V/V158D/E296V/M298Q/K337A-FVIIa, S314E/L305V/V158T/E296V/M298Q/K337A-FVIIa, K316H/L305V/K337A-FVIIa, K316H/L305V/V158D-FVIIa, K316H/L305V/E296V-FVIIa, K316H/L305V/M298Q-FVIIa, K316H/L305V/V158T-FVIIa, K316H/L305V/K337A/V158T-FVIIa, K316H/L305V/K337A/M298Q-FVIIa, K316H/L305V/K337A/E296V-FVIIa, K316H/L305V/K337A/V158D-FVIIa, K316H/L305V/V158D/M298Q-FVIIa, K316H/L305V/V158D/E296V-FVIIa, K316H/L305V/V158T/M298Q-FVIIa, K316H/L305V/V158T/E296V-FVIIa, K316H/L305V/E296V/M298Q-FVIIa, K316H/L305V/V158D/E296V/M298Q-FVIIa, K316H/L305VN158T/E296V/M298Q-FVIIa, K316H/L305V/V158T/K337A/M298Q-FVIIa, K316H/L305V/V158T/E296V/K337A-FVIIa, K316H/L305V/V158D/K337A/M298Q-FVIIa, K316H/L305V/V158D/E296V/K337A-FVIIa, K316H/L305V/V158D/E296V/M298Q/K337A-FVIIa, K316H/L305V/V158T/E296V/M298Q/K337A-FVIIa, K316Q/L305V/K337A-FVIIa, K316Q/L305V/V158D-FVIIa, K316Q/L305V/E296V-FVIIa, K316Q/L305V/M298Q-FVIIa, K316Q/L305V/V158T-FVIIa, K316Q/L305V/K337A/V158T-FVIIa, K316Q/L305V/K337A/M298Q-FVIIa, K316Q/L305V/K337A/E296V-FVIIa, K316Q/L305V/K337A/V158D-FVIIa, K316Q/L305V/V158D/M298Q-FVIIa, K316Q/L305V/V158D/E296V-FVIIa, K316Q/L305V/V158T/M298Q-FVIIa, K316Q/L305V/V158T/E296V-FVIIa, K316Q/L305V/E296V/M298Q-FVIIa, K316Q/L305V/V158D/E296V/M298Q-FVIIa, K316Q/L305V/V158T/E296V/M298Q-FVIIa, K316Q/L305V/V158T/K337A/M298Q-FVIIa, K316Q/L305V/V158T/E296V/K337A-FVIIa, K316Q/L305V/V158D/K337A/M298Q-FVIIa, K316Q/L305V/V158D/E296V/K337A -FVIIa, K316Q/L305V/V158D/E296V/M298Q/K337A-FVIIa, K316Q/L305V/V158T/E296V/M298Q/K337A-FVIIa, and the 407C variants thereof.

**18.** The compound according to any one of the preceding claims, wherein said FVIIa polypeptides are PEGylated.

**19.** A compound which is a conjugate between human serum albumin or a variant thereof and a dimeric or multimeric FVIIa compound according to any one of the preceding claims.

**20.** A pharmaceutical composition comprising a compound according to any one of claims 1-19 and a pharmaceutically acceptable carrier or excipient.

**21.** Use of a compound according to any one of claims 1-19 for the manufacture of a medicament for the treatment of bleeding disorders or bleeding episodes in a subject or for the enhancement of the normal haemostatic system.

**22.** A method for preparation of a compound according to any one of claims 1-19, said method comprising the steps:

a) synthesis and purification of said FVIIa polypeptides;
b) synthesis of said compound by coupling said FVIIa polypeptides;

c) isolation of said dimeric or multimeric FVIIa compound.

**23.** A method for the preparation of a compound according to any one of claims 1-19, said method comprising the steps:

a) synthesis and purification of said FVIIa polypeptides;
b) selective reduction of the cysteine residue on the FVIIa polypeptides which are to be linked;
c) synthesis of said dimeric or multimeric FVIIa compound by coupling said FVIIa polypeptides with reduced cysteine residues in the presence of an activated linker;
d) isolation of said dimeric or multimeric FVIIa compound.

**24.** The method according to claim 23, wherein step b) comprises selective reduction of the cysteine residue on the FVIIa polypeptide by reaction with a redox buffer or a triarylphosphine reducing agent.

**Patentansprüche**

**1.** Dimere oder multimere FVIIa-Verbindung, umfassend mindestens zwei FVIIa Polypeptide, die kovalent verbunden sind, sodass die intrinsische katalytische Aktivität der FVIIa-Polypeptide beibehalten wird, wobei die Verbindung, verglichen mit der biologischen Aktivität der konstituierenden FVIIa-Polypeptide, eine erhöhte biologische Aktivität aufweist, und wobei mindestens zwei FVIIa-Polypeptide über konstruierte Cysteine in den FVIIa-Polypeptiden kovalent verbunden sind.

**2.** Verbindung nach einem der vorangehenden Ansprüche, wobei zwei FVIIa-Polypeptide unter Bildung einer dimeren FVIIa-Verbindung kovalent verbunden sind.

**3.** Verbindung nach einem der vorangehenden Ansprüche, wobei die kovalent verbundenen FVIIa-Polypeptide identische VIIa-Polypeptide sind.

**4.** Verbindung nach einem der vorangehenden Ansprüche, wobei mindestens zwei verschiedene FVIIa-Polypeptide kovalent verbunden sind.

**5.** Verbindung nach einem der vorangehenden Ansprüche, wobei die dimere oder multimere FVIIa-Verbindung eine Verbindungsgruppe zwischen den konstituierenden FVIIa-Polypeptiden umfasst.

**6.** Verbindung nach Anspruch 5, wobei die Verbindungsgruppe die Struktur aufweist, die aus einer Reaktion unter Beteiligung eines zweiwertigen cysteinreaktiven PEGs resultiert.

**7.** Verbindung nach Anspruch 5, wobei die Verbindungsgruppe die Struktur aufweist, die aus der Reaktion unter Beteiligung von Maleimid-PEG-maleimid resultiert.

**8.** Verbindung nach Anspruch 7, wobei das Maleimid-PEG-maleimid ausgewählt ist aus der Gruppe, bestehend aus Maleimid-PEG2kD-Maleimid, Maleimid-PEG3.4kD-maleimid, Maleimid-PEG5kD-maleimid, Maleimid-PEG10kD-maleimid und Maleimid-PEG20kD-maleimid.

**9.** Verbindung nach Anspruch 5, wobei die Verbindungsgruppe eine Aminosäuresequenz umfasst.

**10.** Verbindung nach einem der vorangehenden Ansprüche, wobei das FVIIa-Polypeptid FVIIa 407C ist.

**11.** Verbindung nach einem der vorangehenden Ansprüche, wobei das FVIIa-Polypeptid eine FVIIa 407C-Variante ist.

**12.** Verbindung nach einem der vorangehenden Ansprüche, wobei das FVIIa-Polypeptid FVIIa P406C oder eine FVIIa P406C-Variante ist.

**13.** Verbindung nach einem der vorangehenden Ansprüche, wobei das FVIIa-Polypeptid FVIIa R396C oder eine FVIIa R396C-Variante ist.

**14.** Verbindung nach einem der vorangehenden Ansprüche, wobei das FVIIa-Polypeptid FVIIa Q250C oder eine FVIIa Q250C-Variante ist.

**15.** Verbindung nach einem der vorangehenden Ansprüche, wobei die FVIIa-Polypeptide FVIIa-Varianten mit optimierten GLA-Domänen, z.B. Kombinationen von Y4 (Einfügung) P10Q, K32E, D33F, D33E und A34E sind.

**16.** Verbindung nach einem der vorangehenden Ansprüche, wobei die FVIIa-Polypeptide FVIIa-Varianten mit erhöhter intrinsischer proteolytischer Aktivität, z.B. M298Q-FVIIa, V158D/E296V/M298Q-FVIIa, V158D/E296V/M298Q/K337A-FVIIa, F374Y/L305V-FVIIa, F374Y/L305V/S314E/K337A-FVIIa, F374Y/L305V/S314E-FVIIa, F374Y/L305V/K337A-FVIIa, L305V/K337A-FVIIa, V158D/E296V/M298Q/L305V-FVIIa, V158D/E296V/M298Q/L305V/K337A-FVIIa, V158T/M298Q-FVIIa, E296V/M298Q-FVIIa, V158D/E296V-FVIIa, V158D/M298Q-FVIIa und die 407C-Varianten davon sind.

**17.** Verbindung nach einem der Ansprüche 1 bis 15, wobei die FVIIa-Variante ausgewählt ist aus der Gruppe, bestehend aus L305V-FVIIa, L305V/M306D/D309S-FVIIa, L305I-FVIIa, L305T-FVIIa, F374P-FVIIa, V158T/M298Q-FVIIa, V158D/E296V/M298Q-FVIIa, K337A-FVIIa, M298Q-FVIIa, V158D/M298Q-FVIIa, L305V/K337A-FVIIa, V158D/E296V/M298Q/L305V-FVIIa, V158D/E296V/M298Q/K337A-FVIIa, V158D/E296V/M298Q/L305V/K337A-FVIIa, K157A-FVIIa, E296V-FVIIa, E296V/M298Q-FVIIa, V158D/E296V-FVIIa, V158D/M298K-FVIIa, S336G-FVIIa, L305V/K337A-FVIIa, L305V/V158D-FVIIa, L305V/E296V-FVIIa, L305V/M298Q-FVIIa, L305V/V158T-FVIIa, L305V/K337A/V158T-FVIIa, L305V/K337A/M298Q-FVIIa, L305V/K337A/E296V-FVIIa, L305V/K337A/V158D-FVIIa, L305V/V158D/M298Q-FVIIa, L305V/V158D/E296V-FVIIa, L305V/V158T/M298Q-FVIIa, L305V/V158T/E296V-FVIIa, L305V/E296V/M298Q-FVIIa, L305V/V158D/E296V/M298Q-FVIIa, L305V/V158T/E296V/M298Q-FVIIa, L305V/V158T/K337A/M298Q-FVIIa, L305V/V158T/E296V/K337A-FVIIa, L305V/V158D/K337A/M298Q-FVIIa, L305V/V158D/E296V/K337A-FVIIa, L305V/V158D/E296V/M298Q/K337A-FVIIa, L305V/V158T/E296V/M298Q/K337A-FVIIa, S314E/K316H-FVIIa, S314E/K316Q-FVIIa, S314E/L305V-FVIIa, S314E/K337A-FVIIa, S314E/V158D-FVIIa, S314E/E296V-FVIIa, S314E/M298Q-FVIIa, S314E/V158T-FVIIa, K316H/L305V-FVIIa, K316H/K337A-FVIIa, K316H/V158D-FVIIa, K316H/E296V-FVIIa, K316H/M298Q-FVIIa, K316H/V158T-FVIIa, K316Q/L305V-FVIIa, K316Q/K337A-FVIIa, K316Q/V158D-FVIIa, K316Q/E296V-FVIIa, K316Q/M298Q-FVIIa, K316Q/V158T-FVIIa, S314E/L305V/K337A-FVIIa, S314E/L305V/V158D-FVIIa, S314E/L305V/E296V-FVIIa, S314E/L305V/M298Q-FVIIa, S314E/L305V/V158T-FVIIa, S314E/L305V/K337A/V158T-FVIIa, S314E/L305V/K337A/M298Q-FVIIa, S314E/L308V/K337A/E296V-FVIIa, S314E/L305V/K337A/V158D-FVIIa, S314E/L305V/V158D/M298Q-FVIIa, S314E/L305V/V158D/E296V-FVIIa, S314E/L305V/V158T/M298Q-FVIIa, S314E/L305V/V158T/E296V-FVIIa, S314E/L305V/E296V/M298Q-FVIIa, S314E/L305V/V158D/E296V/M298Q-FVIIa, S314E/L305V/V158T/E296V/M298Q-FVIIa, S314E/L305V/V158T/K337A/M298Q-FVIIa, S314E/L305V/V158T/E296V/K337A-FVIIa, S314E/L305V/V158D/K337A/M298Q-FVIIa, S314E/L305V/V158D/E296V/K337A-FVIIa, S314E/L305V/V158D/E296V/M298Q/K337A-FVIIa, S314E/L305V/V158T/E296V/M298Q/K337A-FVIIa, K316H/L305V/K337A-FVIIa, K316H/L305V/V158D-FVIIa, K316H/L305V/E296V-FVIIa, K316H/L305V/M298Q-FVIa, K316H/L305V/V158T-FVIIa, K316H/L305V/K337A/V158T-FVIIa, K316H/L305V/K337A/M298Q-FVIIa, K316H/L305V/K337A/E296V-FVIIa, K316H/L305V/K337A/V158D-FVIIa, K316H/L305V/V158D/M298Q-FVIIa, K316H/L305V/V158D/E296V-FVIIa, K316H/L305V/V158T/M298Q-FVIIa, K316H/L305V/V158T/E296V-FVIIa, K316H/L305V/E296V/M298Q-FVIIa, K316H/L305V/V158D/E296V/M298Q-FVIIa, K316H/L305V/V158T/E296V/M298Q-FVIIa, K316H/L305V/V158T/K337A/M298Q-FVIIa, K316H/L305V/V158T/E296V/K337A-FVIIa, K316H/L305V/V158D/K337A/M298Q-FVIIa, K316H/L305V/V158D/E296V/K337A-FVIIa, K316H/L305V/V158D/E296V/M298Q/K337A-FVIIa, K316H/L305V/V158T/E296V/M298Q/K337A-FVIIa, K316Q/L305V/K337A-FVIIa, K316Q/L305V/V158D-FVIIa, K316Q/L305V/E296V-FVIIa, K316Q/L305V/M298Q-FVIIa, K316Q/L305V/V158T-FVIIa, K316Q/L305V/K337A/V158T-FVIIa, K316Q/L305V/K337A/M298Q-FVIIa, K316Q/L305V/K337A/E296V-FVIIa, K316Q/L305V/K337A/V158D-FVIIa, K316Q/L305V/V158D/M298Q-FVIIa, K316Q/L305V/V158D/E296V-FVIIa, K316Q/L305V/V158T/M298Q-FVIIa, K316Q/L305V/V158T/E296V-FVIIa, K316Q/L305V/E296V/M298Q-FVIIa, K316Q/L305V/V158D/E296V/M298Q-FVIIa, K316Q/L305V/V158T/E296V/M298Q-FVIIa, K316Q/L305V/V158T/K337A/M298Q-FVIIa, K316Q/L305V/V158T/E296V/K337A-FVIIa, K316Q/L305V/V158D/K337A/M298Q-FVIIa, K316Q/L305V/V158D/E296V/K337A-FVIIa, K316Q/L305V/V158D/E296V/M298Q/K337A-FVIIa, K316Q/L305V/V158T/E296V/M298Q/K337A-FVIIa und den 407C-Varianten davon.

**18.** Verbindung nach einem der vorangehenden Ansprüche, wobei die FVIIa-Polypeptide PEGyliert sind.

**19.** Verbindung, die ein Konjugat zwischen Humanserumalbumin oder einer Variante davon und einer dimeren oder multimeren FVIIa-Verbindung nach einem der vorangehenden Ansprüche ist.

**20.** Arzneimittel, umfassend eine Verbindung nach einem der Ansprüche 1-19 und einen pharmazeutisch verträglichen Träger oder Exzipienten.

**21.** Verwendung einer Verbindung nach einem der Ansprüche 1-19 zur Herstellung eines Medikaments zur Behandlung von Blutungsstörungen oder Blutungsepisoden bei einem Patienten oder zur Verbesserung des normalen hämostatischen Systems.

**22.** Verfahren zur Herstellung einer Verbindung nach einem der Ansprüche 1-19, wobei das Verfahren die folgenden Schritte umfasst:

a) Synthese und Reinigung der FVIIa-Polypeptide;
b) Synthese der Verbindung durch Verbinden der FVIIa-Polypeptide;
c) Isolierung der dimeren oder multimeren FVIIa-Verbindung.

**23.** Verfahren zur Herstellung einer Verbindung nach einem der Ansprüche 1-19, wobei das Verfahren die folgenden Schritte umfasst:

a) Synthese und Reinigung der FVIIa-Polypeptide;
b) Selektive Reduktion des Cysteinrests an den FVIIa-Polypeptiden, die verbunden werden sollen;
c) Synthese der dimeren oder multimeren FVIIa-Verbindung durch Verbinden der FVIIa-Polypeptide mit reduzierten Cysteinresten in der Gegenwart einer aktivierten Verbindungsgruppe;
d) Isolierung der dimeren oder multimeren FVIIa-Verbindung.

**24.** Verfahren nach Anspruch 23, wobei Schritt b) die selektive Reduktion des Cysteinrests am FVIIa-Polypeptid durch Umsetzung mit einem Redoxpuffer oder einem Triarylphosphinreduktionsmittel umfasst.

**Revendications**

**1.** Composé dimère ou multimère du FVIIa comprenant au moins deux polypeptides du FVIIa liés par une liaison covalente de façon à conserver l'activité catalytique intrinsèque des polypeptides du FVIIa, ledit composé ayant une activité biologique accrue par comparaison avec l'activité biologique des polypeptides du FVIIa constituants, et lesdits au moins deux polypeptides du FVIIa étant liés par une liaison covalente par l'intermédiaire de cystéines génétiquement modifiées dans lesdits polypeptides du FVIIa.

**2.** Composé selon l'une quelconque des revendications précédentes, dans lequel deux polypeptides du FVIIa sont liés par une liaison covalente pour former un composé du FVIIa dimère.

**3.** Composé selon l'une quelconque des revendications précédentes, dans lequel lesdits polypeptides du FVIIa liés par une liaison covalente sont des polypeptides du FVIIa identiques.

**4.** Composé selon l'une quelconque des revendications précédentes, dans lequel au moins deux polypeptides du FVIIa différents sont liés par une liaison covalente.

**5.** Composé selon l'une quelconque des revendications précédentes, dans lequel ledit composé dimère ou multimère du FVIIa comprend un lieur entre les polypeptides du FVIIa constituants.

**6.** Composé selon la revendication 5, dans lequel ledit lieur a la structure qui résulte d'une réaction impliquant un PEG divalent réactif vis-à-vis de la cystéine.

**7.** Composé selon la revendication 5, dans lequel ledit lieur a la structure qui résulte d'une réaction impliquant un maléimide-PEG-maléimide.

**8.** Composé selon la revendication 7, dans lequel ledit maléimide-PEG-maléimide est choisi dans le groupe consistant en le maléimide-PEG2kD-maléimide, le maléimide-PEG3,4kD-maléimide, le maléimide-PEG5kD-maléimide, le maléimide-PEGlOkD-maléimide et le maléimide-PEG20kD-maléimide.

**9.** Composé selon la revendication 5, dans lequel ledit lieur comprend une séquence d'acides aminés.

**10.** Composé selon l'une quelconque des revendications précédentes, dans lequel ledit polypeptide du FVIIa est le FVIIa 407C.

**11.** Composé selon l'une quelconque des revendications précédentes, dans lequel ledit polypeptide du FVIIa est un variant du FVIIa 407C.

**12.** Composé selon l'une quelconque des revendications précédentes, dans lequel ledit polypeptide du FVIIa est le FVIIa P406C ou un variant du FVIIa P406C.

**13.** Composé selon l'une quelconque des revendications précédentes, dans lequel ledit polypeptide du FVIIa est le FVIIa R396C ou un variant du FVIIa du R396C.

**14.** Composé selon l'une quelconque des revendications précédentes, dans lequel ledit polypeptide du FVIIa est le FVIIa Q250C ou un variant du FVIIa Q250C.

**15.** Composé selon l'une quelconque des revendications précédentes, dans lequel lesdits polypeptides du FVIIa sont des variants du FVIIa ayant des domaines GLA optimisés, p.ex. des combinaisons de Y4 (insertion), de P10Q, de K32E, de D33F, de D33E et de A34E.

**16.** Composé selon l'une quelconque des revendications précédentes, dans lequel lesdits polypeptides du FVIIa sont des variants du FVIIa ayant une activité protéolytique intrinsèque accrue, p.ex. M298Q FVIIa, V158D/E296V/M298Q FVIIa, V158D/E296V/M296Q/K337A FVIIa, F374Y/L305V-FVIIa, F374Y/L305V/S314E/K337A-FVIIa, F374Y/L305V/S314E-FVIIa, F374Y/L305V/K337A-FVIIa, L305V/K337A-FVIIa, V158D/E296V/M2980/L305V-FVIIa, V158D/E296V/M298Q/L305V/K337A-FVIIa, V158T/M298Q-FVIIa, E296V/M298Q-FVIIa, V158D/E296V-FVIIa, V158D/M298Q-FVIIa et les variants 407C de ceux-ci.

**17.** Composé selon l'une quelconque des revendications 1 à 15, dans lequel ledit variant du FVIIa est choisi dans le groupe consistant en L305V-FVIIa, L305V/M306D/D309S-FVIIa, L305I-FVIIa, L305T-FVIIa, F374P-FVIIa, V158T/M298Q-FVIIa, V158D/E296V/M298Q-FVIIa, K337A-FVIIa, M298Q-FVIIa, V158D/M298Q-FVIIa, L305V/K337A-FVIIa, V158D/E296V/M298Q/L305V-FVIIa, V158D/E296V/M298Q/K337A-FVIIa, V158D/E296V/M298Q/L305V/K337A-FVIIa, K157A-FVIIa, E296V-FVIIa, E296V/M298Q-FVIIa, V158D/E296V-FVIIa, V158D/M298K-FVIIa, S336G-FVIIa, L305V/K337A-FVIIa, L305V/V158D-FVIIa, L305V/E296V-FVIIa, L305V/M298Q-FVIIa, L305V/V158T-FVIIa, L305V/K337A/V158T-FVIIa, L305V/K337A/M298Q-FVIIa, L305V/K337A/E296V-FVIIa. L305V/K337A/V158D-FVIIa, L305V/V/158D/M298Q-FVIIa, L305V/V158D/E296V-FVIIa, L305V/V158T/M298Q-FVIIa, L305V/V158T/E296V-FVIIa, L305V/E296V/M298Q-FVIIa, L305V/V158D/E296V/M298Q-FVIIa, L305V/V158T/E296V/M298Q-FVIIa, L305V/V158T/K337A/M298Q-FVIIa, L305V/V158T/E296V/K337A-FVIIa, L305V/V158D/K337A/M298Q-FVIIa, L305V/V158D/E296V/K337A-FVIIa, L305V/V158D/E296V/M298Q/K337A-FVIIa, L305V/V158T/E296V/M298Q/K337A-FVIIa, S314E/K316H-FVIIa, S314E/K316Q-FVIIa, S314E/L305V-FVIIa, S314E/K337A-FVIIa, S314E/V156D-FVIIa, S314E/E296V-FVIIa, S314E/M298Q-FVIIa, S314E/V158T-FVIIa, K316H/L305V-FVIIa, K316H/K337A-FVIIa, K316H/V158D-FVIIa, K316H/E296V-FVIIa, K316H/M2980-FVIIa, K316H/V158T-FVIIa, K316Q/L305V-FVIIa, K316Q/K337A-FVIIa, K316Q/V158D-FVIIa, K316Q/E296V-FVIIa, K316Q/M298Q-FVIIa, K316Q/V158T-FVIIa, S314E/L305V/K337A-FVIIa, S314E/L305V/V158D-FVIIa, S314E/L305V/E296V-FVIIa, S314E/L305V/M298Q-FVIIa, S314E/L305V/V158T-FVIIa, S314E/L305V/K337A/V158T-FVIIa, S314E/L305V/K337A/M298Q-FVIIa, S314E/L305V/K337A/E296V-FVIIa, S314E/L305V/K337A/V158D-FVIIa, S314E/L305V/V158D/M298Q-FVIIa, S314E/L305V/V158D/E296V-FVIIa, S314E/L305V/V158T/M298Q-FVIIa, S314E/L305V/V158T/E296V-FVIIa, S314E/L305V/E296V/M298Q-FVIIa, S314E/L305V/V158D/E296V/M298Q-FVIIa, S314E/L305V/V158T/E296V/M298Q-FVIIa, S314E/L305V/V158T/K337A/M298Q-FVIIa, S314E/L305V/V158T/E296V/K337A-FVIIa, S314E/L305V/V158D/K337A/M298Q-FVIIa, S314E/L305V/V158D/E296V/K337A-FVIIa, S314E/L305V/V158D/E296V/M298Q/K337A-FVIIa, S314E/L305V/V158T/E296V/M298Q/K337A-FVIIa, K316H/L305V/K337A-FVIIa, K316H/L305V/V158D-FVIIa, K316H/L305V/E296V-FVIIa, K316H/L305V/M298Q-FVIIa, K316H/L305V/V158T-FVIIa, K316H/L305V1K337A/V158T-FVIIa, K316H/L305V/K337A/M2980-FVIIa, K316H/L305V/K337A/E296V-FVIIa, K316H/L305V/K337A/V158D-FVIIa, K316H/L305V/V158D/M298Q-FVIIa, K316H/L3075V/V158D/E296V-FVIIa, K316H/L305V/V158T/M298Q-FVIIa, K316H/L305V/V158T/E296V-FVIIa, K316H/L305V/E296V/M298Q-FVIIa, K316H/L305V/V158D/E296V/M298Q-FVIIa, K316H/L305V/V158T/E296V/M298Q-FVIIa, K316H/L305V/V158T/K337A/M298Q-FVIIa, K316H/L305V/V158T/E296V/K337A-FVIIa, K316H/L305V/V158D/K337A/M298Q-FVIIa, K318H/L305V/V158D/E296/K337A-FVIIa,

K316H/L305V/V158D/E296V/M298Q/K337A-FVIIa, K316H/L305VN158T/E296V/M298Q/K337A-FVIIa, K316Q/L305V/K337A-FVIIa, K316Q/L305V/V1580-FVIIa, K316Q/L305V/E296V-FVIIa, K316Q/L305V/M298Q-FVIIa, K316Q/L305V/V158T-FVIIa, K316Q/L305V/K337A/V158T-FVIIa, K316Q/L305V/K337A/M298Q-FVIIa, K316Q/L305V/K337A/E296V-FVIIa, K316Q/L305V/K337A/V158D-FVIIa, K316Q/L305V/V158D/M298Q-FVIIa, K316Q/L305V/V158D/E296V-FVIIa, K316Q/L305V/V158T/M2980-FVIIa, K316Q/L305V/V158T/E296V-FVIIa, K316Q/L305V/E296V/M298Q-FVIIa, K316Q/L305V/V158D/E296V/M298Q-FVIIa, K316Q/L305V/V158T/E296V/M298Q-FVIIa, K316Q/L305V/V158T/K337A/M298Q-FVIIa, K316Q/L305V/V158T/E296V/K337A-FVIIa, K316Q/L305V/V158D/K337A/M298Q-FVIIa, K316Q/L305V/V158D/E296V/K337A-FVIIa, K316Q/L305V/V158DE296V/M298Q/K337A-FVIIa, K316Q/L305V/V158T/E296V/M298Q/K337A-FVIIa, et les variants 407C de Ceux-Ci.

**18.** Composé selon l'une quelconque des revendications précédentes, dans lequel lesdits polypeptides du FVIIa sont PEGylés.

**19.** Composé qui est un conjugué entre la sérum-albumine humaine ou un variant de cette dernière et un composé dimère ou multimère du FVIIa selon l'une quelconque des revendications précédentes.

**20.** Composition pharmaceutique comprenant un composé selon l'une quelconque des revendications 1 à 19 et un support ou un excipient pharmaceutiquement acceptable.

**21.** Utilisation d'un composé selon l'une quelconque des revendications 1 à 19 pour fabriquer un médicament destiné au traitement des troubles hémostatiques ou des épisodes hémorragiques chez un sujet, ou pour le renforcement du système hémostatique normal.

**22.** Procédé de préparation d'un composé selon l'une quelconque des revendications 1 à 19, ledit procédé comprenant les étapes suivantes :

a) synthèse et purification desdits polypeptides du FVIIa ;
b) synthèse dudit composé par couplage desdits polypeptides du FVIIa ;
c) isolement dudit composé dimère ou multimère du FVIIa.

**23.** Procédé pour la préparation d'un composé selon l'une quelconque des revendications 1 à 19, ledit procédé comprenant les étapes suivantes :

a) synthèse et purification desdits polypeptides du FVIIa ;
b) réduction sélective du résidu de cystéine sur les polypeptides du FVIIa qui doivent être liés ;
c) synthèse dudit composé dimère ou multimère du FVIIa par couplage desdits polypeptides du FVIIa à des résidus de cystéine réduits, en présence d'un lieur activé ;
d) isolement dudit composé dimère ou multimère du FVIIa.

**24.** Procédé selon la revendication 23, dans lequel l'étape b) comprend la réduction sélective du résidu de cystéine sur le polypeptide du FVIIa par réaction avec un tampon rédox ou un agent réducteur triarylphosphine.

Figure 1

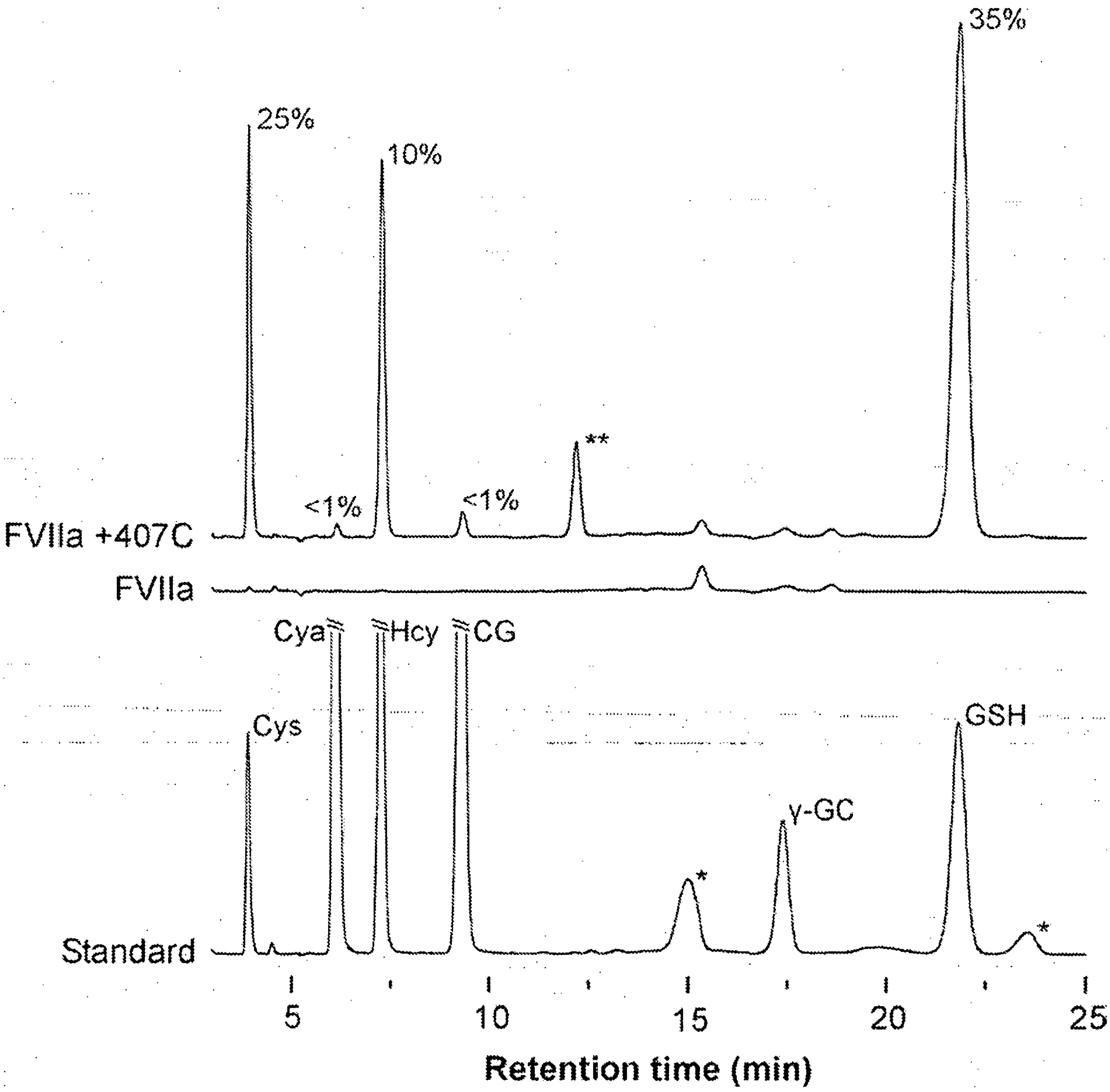
35%
25%
10%
<1%
<1%
**
FVIIa +407C
FVIIa
Cya
Hcy
CG
Cys
GSH
γ-GC
*
*
Standard
5
10
15
20
25
Retention time (min)

**Figure 2**

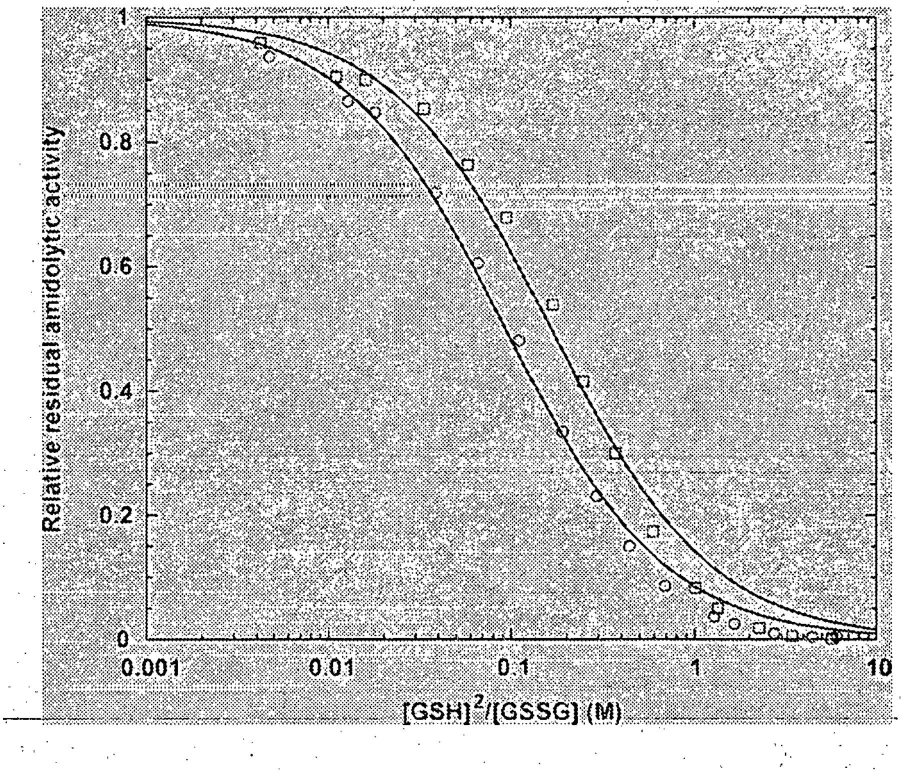

Relative residual amidolytic activity
1
0.8
0.6
0.4
0.2
0
0.001
0.01
0.1
1
10
[GSH]$^2$/[GSSG] (M)

**Figure 3**

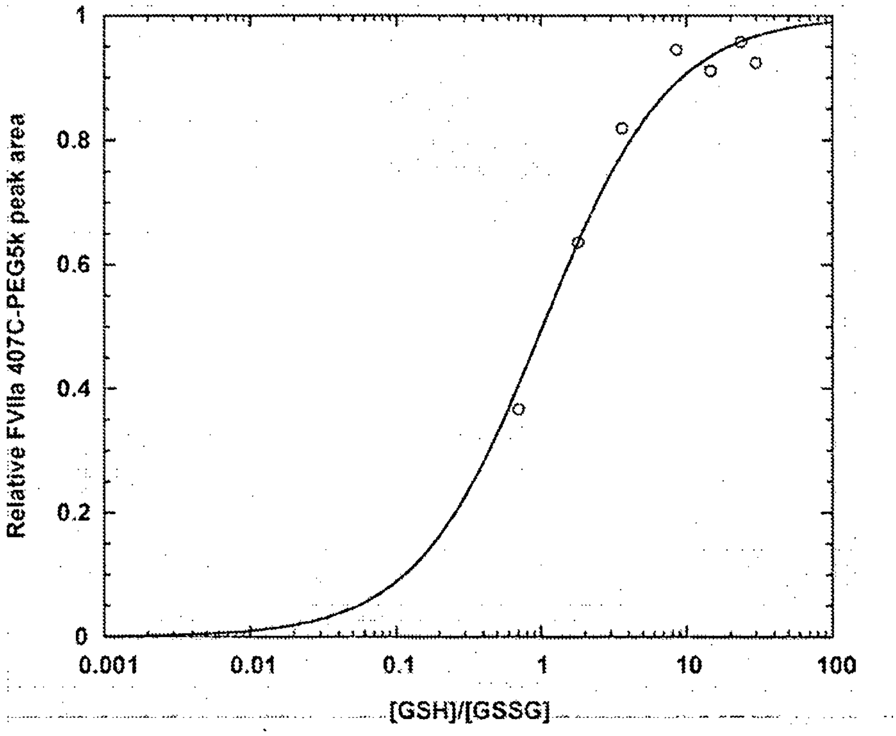
Relative FVIIa 407C-PEG5k peak area
1
0.8
0.6
0.4
0.2
0
0.001
0.01
0.1
1
10
100
[GSH]/[GSSG]

**Figure 4**

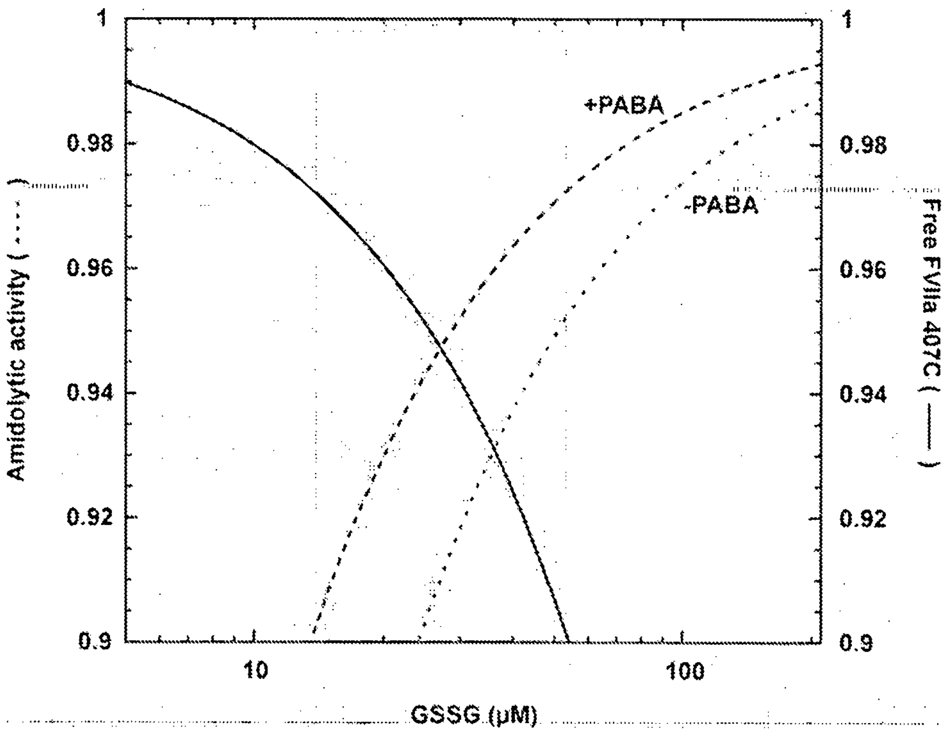

1
0.98
0.96
0.94
0.92
0.9
10
100
GSSG (µM)
Amidolytic activity ( - - - - )
Free FVIIa 407C ( —— )
+PABA
-PABA

**Figure 5**

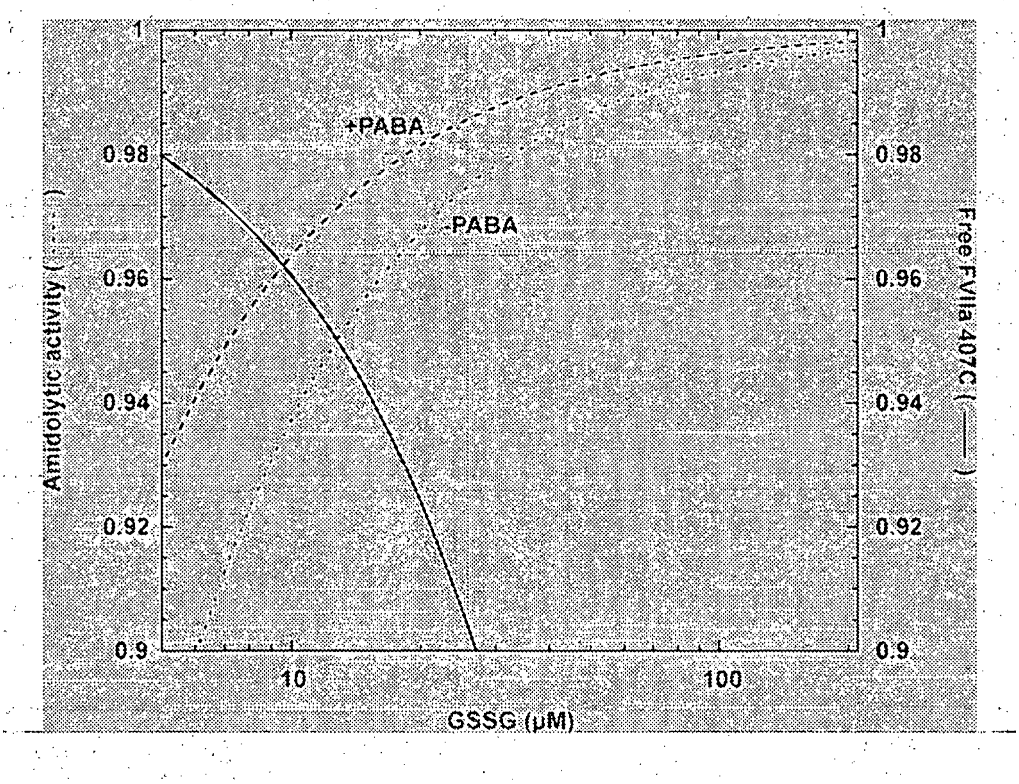
+PABA
-PABA
1
0.98
0.96
0.94
0.92
0.9
10
100
GSSG (μM)
Amidolytic activity (-----)
Free FVIIa 407C (——)

**Figure 6**

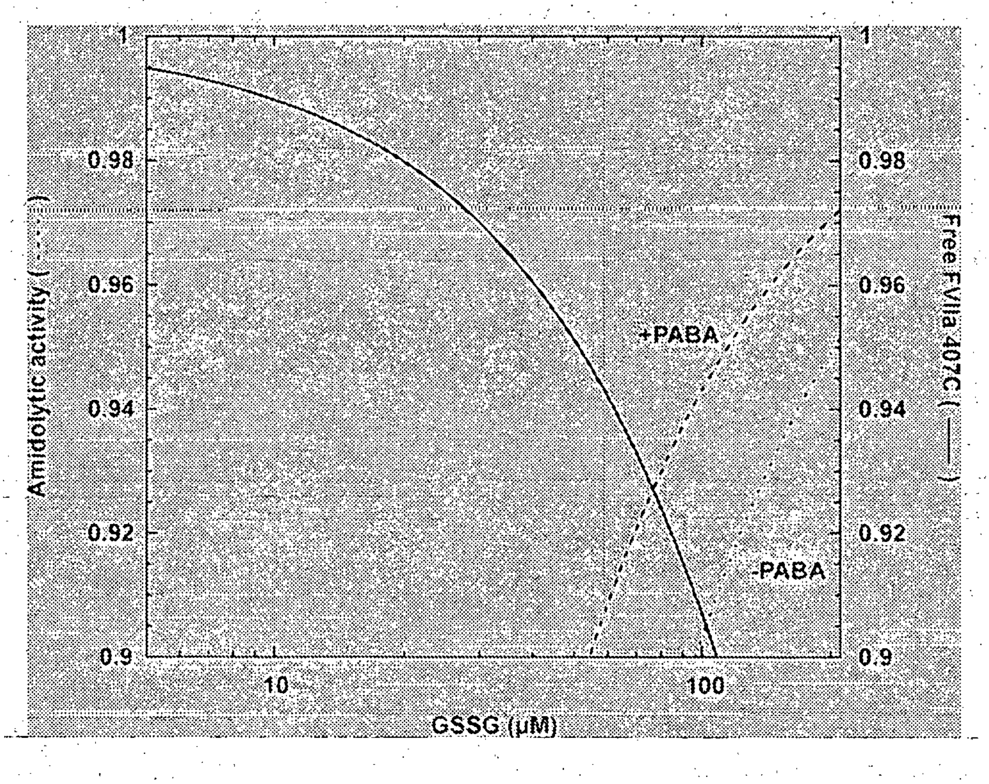
1
0.98
0.96
0.94
0.92
0.9
Amidolytic activity (------)
Free FVIIa 407C (——)
+PABA
-PABA
10
100
GSSG (µM)

**Figure 7**

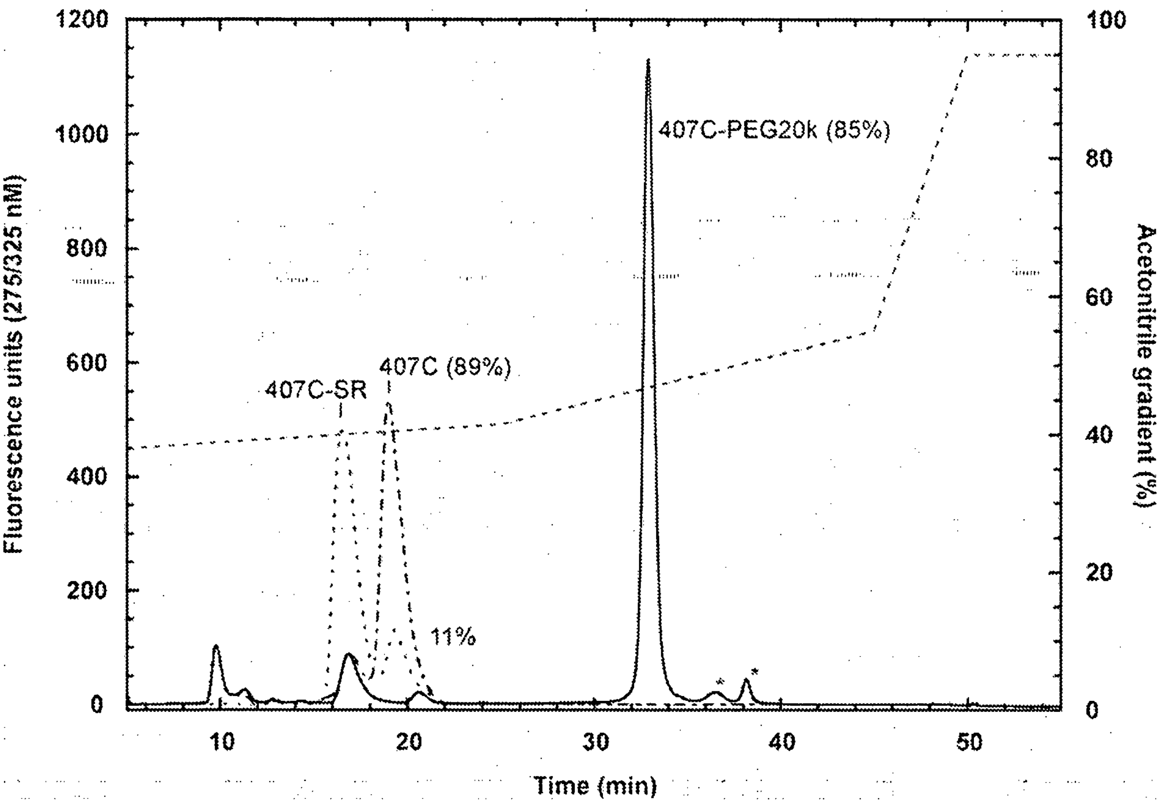

1200
1000
800
600
400
200
0
Fluorescence units (275/325 nM)
100
80
60
40
20
0
Acetonitrile gradient (%)
10
20
30
40
50
Time (min)
407C-PEG20k (85%)
407C (89%)
407C-SR
11%
*
*

**Figure 8**

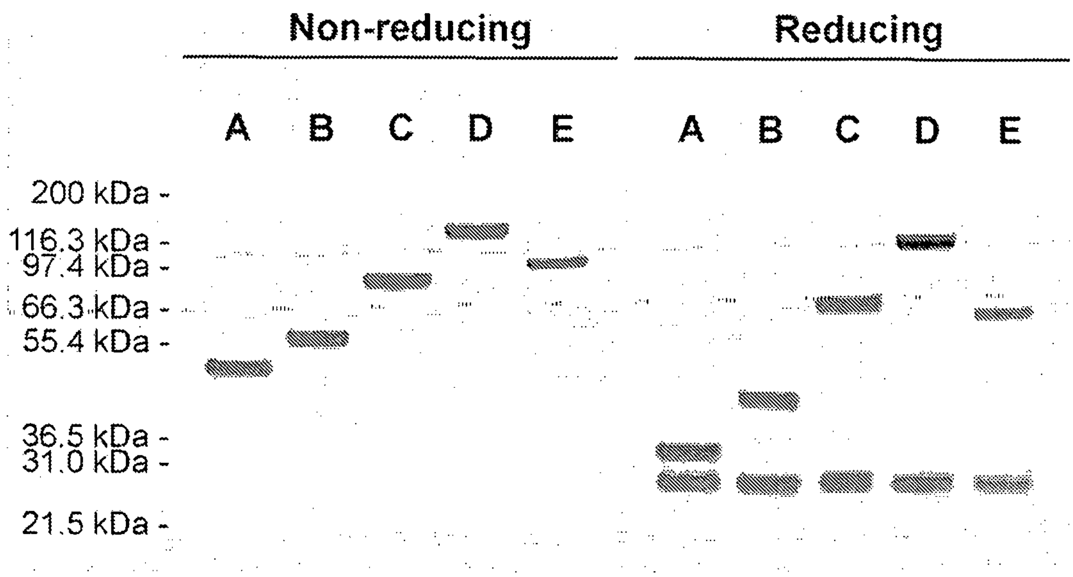
Non-reducing
Reducing
A B C D E
A B C D E
200 kDa -
116.3 kDa -
97.4 kDa -
66.3 kDa -
55.4 kDa -
36.5 kDa -
31.0 kDa -
21.5 kDa -

**Figure 9**

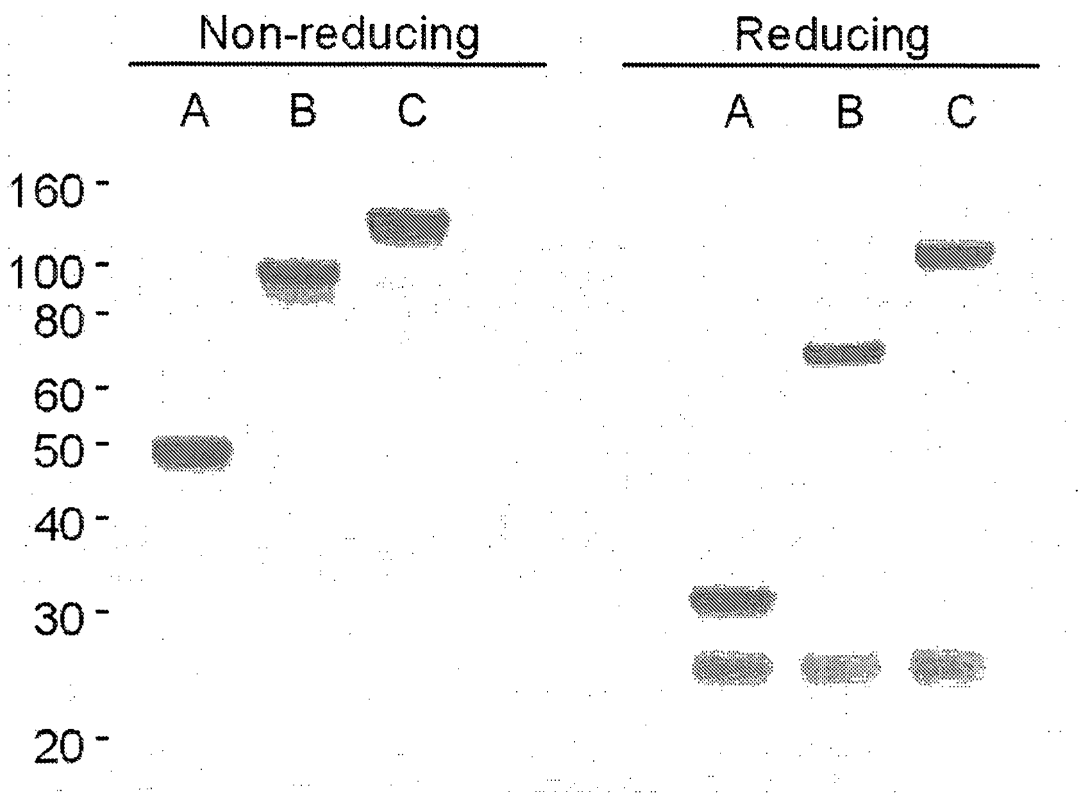

**Figure 10**

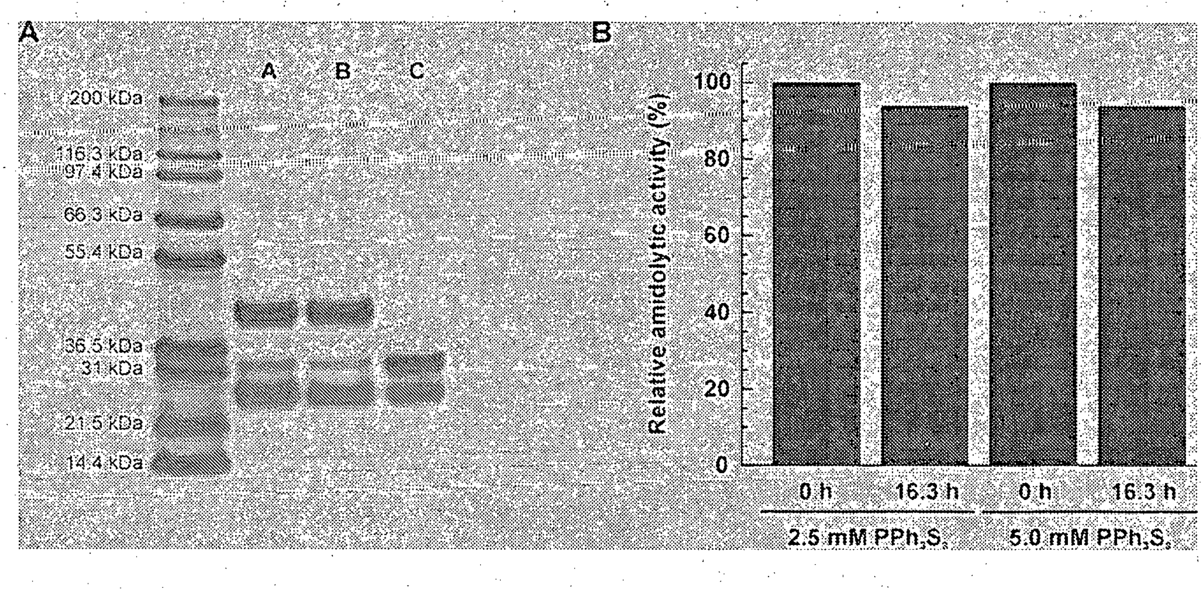
A
A
B
C
200 kDa
116.3 kDa
97.4 kDa
66.3 kDa
55.4 kDa
36.5 kDa
31 kDa
21.5 kDa
14.4 kDa
B
Relative amidolytic activity (%)
100
80
60
40
20
0
0 h
16.3 h
0 h
16.3 h
2.5 mM PPh3S
5.0 mM PPh3S

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02077218 A1
- WO 0158935 A2
- WO 03076461 A
- SE 9501285 A
- WO 0331464 A
- US 20040043446 A
- US 20040063911 A
- US 20040142856 A
- US 20040137557 A
- US 20040132640 A
- WO 0104287 A
- US 20030165996 A
- WO 0158935 A
- WO 0393465 A
- WO 0202764 A
- US 20030211094 A
- US 5580560 A
- DK 0200189 W
- WO 02077218 A
- WO 0238162 A
- WO 9920767 A
- US 6017882 A
- US 6747003 B
- US 20030100506 A
- WO 0066753 A
- US 20010018414 A
- US 2004220106 A
- US 200131005 B
- US 6762286 B
- US 6693075 B
- US 6806063 B
- US 20030096338 A
- WO 04029091 A
- WO 04083361 A
- WO 04111242 A
- WO 04108763 A
- WO 0183725 A
- WO 0222776 A
- DK 0200635 W
- WO 03027147 A
- DK PA200201423
- WO 04029090 A
- DK PA200101627
- JP 2001061479 B
- WO 05016365 A3
- EP 1162194 A1
- EP 1270551 A1
- WO 9013540 A
- US 5932462 A
- US 5643575 A
- US 5824778 A
- US 5476653 A
- WO 9732607 A
- EP 229108 A
- EP 402378 A
- US 4902502 A
- US 5281698 A
- US 5122614 A
- US 5219564 A
- WO 9216555 A
- WO 9404193 A
- WO 9414758 A
- WO 9417039 A
- WO 9418247 A
- WO 9428024 A
- WO 9500162 A
- WO 9511924 A
- WO 9513090 A
- WO 9533490 A
- WO 9600080 A
- WO 9718832 A
- WO 9841562 A
- WO 9848837 A
- WO 9932134 A
- WO 9932139 A
- WO 9932140 A
- WO 9640791 A
- WO 9832466 A
- WO 9506058 A
- EP 439508 A
- WO 9703106 A
- WO 9621469 A
- WO 9513312 A
- EP 921131 A
- US 5736625 A
- WO 9805363 A
- EP 809996 A
- US 5629384 A
- WO 9641813 A
- WO 9607670 A
- US 5473034 A
- US 5516673 A
- EP 605963 A
- US 5382657 A
- EP 510356 A
- EP 400472 A
- EP 183503 A

- EP 154316 A
- US 5985265 A
- WO 9955377 A

**Non-patent literature cited in the description**


- **LINO et al.** *Arch. Biochem. Biophys,* 1998, vol. 352, 182-192
- **MOLLERUP et al.** *Biotechnol. Bioeng,* 1995, vol. 48, 501-505
- **KORNFELT et al.** *Arch. Biochem. Biophys.,* 1999, vol. 363, 43-54
- **R. F. TAYLOR.** Protein immobilisation. Fundamental and applications. Marcel Dekker, 1991
- **S. S. WONG.** Chemistry of Protein Conjugation and Crosslinking. CRC Press, 1992
- **G. T. HERMANSON et al.** Immobilized Affinity Ligand Techniques. Academic Press, 1993
- **BOCK,P.E.** Active-site-selective labeling of blood coagulation proteinases with fluorescence probes by the use of thioester peptide chloromethyl ketones. I. Specificity of thrombin labeling. *J Biol Chem,* 1992, vol. 267, 14963-14973
- **CHAU,M.H. ; NELSON,J.W.** Direct measurement of the equilibrium between glutathione and dithiothreitol by high performance liquid chromatography. *FEBS Lett.,* 1991, vol. 291, 296-298
- **FERNANDES, A.P. ; HOLMGREN, A.** Glutaredoxins: glutathione-dependent redox enzymes with functions far beyond a simple thioredoxin backup system. *Antioxid.Redox.Signal.,* 2004, vol. 6, 63-74
- **FRESKGARD,P.O. ; OLSEN,O.H. ; PERSSON,E.** Structural changes in factor VIIa induced by Ca2+ and tissue factor studied using circular dichroism spectroscopy. *Protein Sci,* 1996, vol. 5, 1531-1540
- **GILBERT,H.F.** Thiol/disulfide exchange equilibria and disulfide bond stability. *Methods Enzymol.,* 1995, vol. 251, 8-28
- **GRANT,C.** MicroReview: Role of the glutathione/glutaredoxin and thioredoxin systems in yeast growth and response to stress conditions. *Mol. Microbiol.,* 2001, vol. 39, 533-541
- **GRIMBERG,J. ; MAGUIRE,S. ; BELLUSCIO,L.** A simple method for the preparation of plasmid and chromosomal E. coli DNA. *Nucleic Acids Res,* 1989, vol. 17, 8893
- **HARVEY et al.** *J. Biol. Chem.,* 2003, vol. 278, 8363-8369
- **HEMKER, H. C. ; GIESEN, P. L. ; RAMJEE, M. ; WAGENVOORD, R. ; BEGUIN, S..** The thrombogram: monitoring thrombin generation in platelet-rich plasma. *Thromb.Haem.,* 2000, vol. 83 (4), 589-591
- **HEMKER, H. C. ; BEGUIN, S.** Phenotyping the clotting system. *Thromb.Haem.,* 2000, vol. 84 (5), 747-751
- **HIGASHI,S. ; MATSUMOTO,N. ; IWANAGA,S.** Conformation of factor VIIa stabilized by a labile disulfide bond (Cys-310-Cys-329) in the protease domain is essential for interaction with tissue factor. *J. Biol. Chem.,* 1997, vol. 272, 25724-25730
- **HOLMGREN,A. ; ÅSLUND,F.** Glutaredoxin. *Method Enzymol.,* 1995, vol. 252, 283-292
- **HOFFMAN,C.S. ; WINSTON,F.** A ten-minute DNA preparation from yeast efficiently releases autonomous plasmids for transformation of Escherichia coli. *Gene,* 1987, vol. 57, 267-272
- **LOFERER,H. ; WUNDERLICH,M. ; HENNECKE,H. ; GLOCKSHUBER,R.** A bacterial thioredoxin-like protein that is exposed to the periplasm has redox properties comparable with those of cytoplasmic thioredoxins. *J. Biol. Chem.,* 1995, vol. 270, 26178-26183
- **LUIKENHUIS,S. ; PERRONE,G. ; DAWES,I.W. ; GRANT,C.M.** The yeast Saccharomyces cerevisiae contains two glutaredoxin genes that are required for protection against reactive oxygen species. *Mol. Biol. Cell,* 1998, vol. 9, 1081-1091
- **LUNDBERG,M. ; JOHANSSON,C. ; CHANDRA,J. ; ENOKSSON,M. ; JACOBSSON,G. ; LJUNG,J. ; JOHANSSON,M. ; HOLMGREN,A.** Cloning and expression of a novel human glutaredoxin (Grx2) with mitochondrial and nuclear isoforms. *J Biol Chem,* 2001, vol. 276, 26269-26275
- **NELSESTUEN et al.** *J. Biol. Chem.,* 2001, vol. 276, 39825-39831
- **RODRIGUEZ-MANZANEQUE,M.T. ; ROS,J. ; CABISCOL,E. ; SORRIBAS,A. ; HERRERO,E.** Grx5 glutaredoxin plays a central role in protection against protein oxidative damage in Saccharomyces cerevisiae. *Mol Cell Biol,* 1999, vol. 19, 8180-8190
- **OE,T. ; OHYAGI,T. ; NAGANUMA,A.** Determination of γ-glutamylglutathione and other low-molecular-mass thiol compounds by isocratic high-performance liquid chromatography with fluorimetric detection. *J. Chrom. B,* 1998, vol. 708, 285-289
- **OSTERGAARD,H. ; TACHIBANA,C. ; WINTHER,J.R.** Monitoring disulfide bond formation in the eukaryotic cytosol. *J Cell Biol,* 2004, vol. 166, 337-345
- **OWENIUS,R.I ; OSTERLUND,M. ; LINDGREN,M. ; SVENSSON,M. ; OLSEN,O.H. ; PERSSON,E. ; FRESKGÅRD,P.-O. ; CARLSSON,U.** Properties of spin and fluorescent labels at a receptor-ligand interface. *Biophys. J.,* 1999, vol. 77, 2237-2250

- **PADILLA,C.A. ; MARTINEZ-GALISTEO,E. ; BARCENA,J.A. ; SPYROU,G. ; HOLMGREN,A.** Purification from placenta, amino acid sequence, structure comparisons and cDNA cloning of human glutaredoxin. *Eur J Biochem,* 1995, vol. 227, 27-34
- **PERSSON et al.** *J. Biol. Chem.,* 2001, vol. 276, 29195-29199
- **PERSSON et al.** *Proc. Natl. Acad. Sci.,* 2001, vol. 98, 13583-13588
- **PERSSON et al.** *Eur. J. Biochem.,* 2002, vol. 379, 497-503
- **PERSSON,E. ; BAK,H. ; OSTERGAARD,A. ; OLSEN,O.H.** Augmented intrinsic activity of Factor VIIa by replacement of residues 305, 314, 337 and 374: evidence of two unique mutational mechanisms of activity enhancement. *Biochem J,* 2004, vol. 379, 497-503
- **PERSSON,E. ; NIELSEN,L.S.** Site-directed mutagenesis but not gamma-carboxylation of Glu-35 in factor VIIa affects the association with tissue factor. *FEBS Lett,* 1996, vol. 385, 241-243
- **RIDDLES,P.W. ; BLAKELEY,R.L. ; ZERNER,B.** Ellman's reagent: 5,5'-dithiobis(2-nitrobenzoic acid)--a reexamination. *Anal. Biochem.,* 1979, vol. 94, 75-81
- **SAMBROOK,J. ; FRITSCH,E.F. ; MANIATIS,T.** Molecular Cloning: A Laboratory Manual. Cold Spring Harbor Laboratory, 1989
- **SHAH et al.** *Proc. Natl. Acad. Sci.,* 1998, vol. 95, 4229-4234
- **SICHLER,K. ; BANNER,D.W. ; D'ARCY,A. ; HOPFNER,K.P. ; HUBER,R. ; BODE,W. ; KRESSE,G.B. ; KOPETZKI,E. ; BRANDSTETTER,H.** Crystal structures of uninhibited factor VIIa link its cofactor and substrate-assisted activation to specific interactions. *J. Mol. Biol.,* 2002, vol. 322, 591-603
- **SOEJIMA et al.** *J. Biol. Chem.,* 2002, vol. 277, 49027
- **TAKAHASHI,N. ; CREIGHTON,T.E.** On the reactivity and ionization of the active site cysteine residues of Escherichia coli thioredoxin. *Biochemistry,* 1996, vol. 35, 8342-8353
- **THIM,L. ; BJOERN,S. ; CHRISTENSEN,M. ; NICOLAISEN,E.M. ; LUND-HANSEN,T. ; PEDERSEN,A.H. ; HEDNER,U.** Amino acid sequence and posttranslational modifications of human factor VIIa from plasma and transfected baby hamster kidney cells. *Biochemistry,* 1988, vol. 27, 7785-7793
- **WANG, E.C.W. ; HUNG, S.-H. ; CAHOON,M. ; HEDSTROM,L.** The role of Cys191-Cys220 disulfide bond in trypsin: new targets for engineering substrate specificity. *Protein Engineering,* 1997, vol. 10, 405-411
- **YANG,Y. ; JAO,S. ; NANDURI,S. ; STARKE,D.W. ; MIEYAL,J.J. ; QIN,J.** Reactivity of the human thioltransferase (glutaredoxin) C7S, C25S, C78S, C82S mutant and NMR solution structure of its glutathionyl mixed disulfide intermediate reflect catalytic specificity. *Biochemistry,* 1998, vol. 37, 17145-17156